(19) Europäisches Patentamt
European Patent Office
Office européen des brevets

(11) EP 2 305 272 A1

# (12) EUROPEAN PATENT APPLICATION

(43) Date of publication:
**06.04.2011 Bulletin 2011/14**

(51) Int Cl.:
***A61K 31/7048*** *(2006.01)* ***A61K 31/353*** *(2006.01)*
***A61K 47/00*** *(2006.01)* ***A61P 17/00*** *(2006.01)*

(21) Application number: **10194495.7**

(22) Date of filing: **17.02.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **28.04.2004 US 521457 P**

(62) Document number(s) of the earlier application(s) in accordance with Art. 76 EPC:
**05723182.1 / 1 740 191**

(71) Applicants:
- **Brigham Young University**
  **Provo, UT 84602-6844 (US)**
- **Children's Hospital Medical Center**
  **Cincinnati, Ohio 45229 (US)**
- **THE COLORADO STATE UNIVERSITY RESEARCH FOUNDATION**
  **Fort Collins, CO 80522 (US)**

(72) Inventors:
- **Lephart, Edwin Douglas**
  **Orem, UT 84097 (US)**
- **Lund, Trent D**
  **St. Charles, IL 60174 (US)**
- **Setchell, Kenneth David Reginald**
  **Cincinnati, OH 45227 (US)**
- **Handa, Robert J**
  **Fort Collins, CO 80526 (US)**

(74) Representative: **Blance, Stephen John**
**D Young & Co LLP**
**120 Holborn**
**London EC1N 2DY (GB)**

Remarks:
This application was filed on 10-12-2010 as a divisional application to the application mentioned under INID code 62.

## (54) Use of equol for treating skin diseases

(57) Equol (7-hydroxy-3(4'hydroxyphenyl)-chroman), the major metabolite of the phytoestrogen daidzein, specifically binds and blocks the hormonal action of 5adihydrotestosterone (DHT) in vitro and in vivo. Equol can bind circulating free DHT and sequester it from the androgen receptor, thus altering growth and physiological hormone responses that are regulated by androgens. These data suggest a novel model to explain equol's biological properties. The significance of equol's ability to specifically bind and sequester DHT from the androgen receptor have important ramifications in health and disease and may indicate a broad and important usage for equol in the treatment and prevention of androgen-mediated pathologies of skin and hair. Thus, equol can specifically bind DHT and prevent DHT's biological actions in physiological and pathophysiological processes affecting skin and hair.

Figure 1

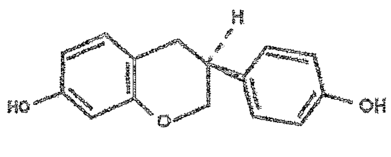

S-equol

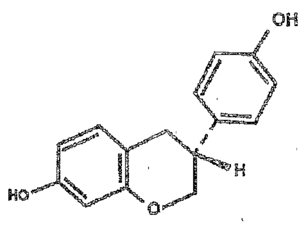

R-equol



EP 2 305 272 A1

## Description

GOVERNMENT INTERESTS

This invention was made with Government support under Grant # NS39951, awarded by the National Institute of Health (NIH), and Grants # NRI 2002-00798 and # NRI 2004-01811 awarded by the U.S. Dept. of Agriculture (USDA).
The Government has certain rights in this invention.

BACKGROUND OF THE INVENTION

This invention relates equol and its mechanism of action and use as a therapeutic compound for treating and preventing physiological and pathophysiological conditions mediated by androgens.

In recent years phytoestrogens have received increased investigative attention due to their potential protective effects against age-related diseases (e.g. cardiovascular disease and osteoporosis) and hormone-dependent cancers (i.e., breast and prostate cancer). There are three main classifications of phytoestrogens: 1) isoflavones (derived principally from soybeans), 2) lignans (found in flaxseed in large quantities) and 3) coumestans (derived from sprouting plants like alfalfa). Of these three main classifications, human consumption of isoflavones has the largest impact due to its availability and variety in food products containing soy. Of the isoflavones, genistein and daidzein are thought to exert the most potent estrogenic hormone activity and thus most attention has been directed toward these molecules (Knight et al, Obstet Gyneco, 187:897-904, (1996); Setchell, KDR. Am J Clin Nutr, 129:1333S-1346S (1998); Kurzer et al, Annu Rev Nutr, 17:353-381(1997)). However, these isoflavone molecules do not exist at high levels in their biologically active form in soy foods, but rather are at high abundance in a precursor form. For example, genistin, the precursor of genistein, is the glycosidic form that contains a carbohydrate portion of the molecule. Additionally, malonylglucoside and acetylglucoside forms also are found. These conjugates are metabolized in the gastrointestinal (GI) tract by intestinal bacteria, which hydrolyze the carbohydrate moiety to the biologically active phytoestrogen, genistein. The same metabolic step occurs for the aglycone daidzein, which is converted from the glycosidic form daidzin. Diadzein is then further metabolized to equol in an "equol-producing" mammal, which is then found in the plasma of an equol-producing individual. Equol is not normally present in the urine of most healthy human adults unless soy is consumed. The formation of equol in vivo is exclusively dependent on intestinal microflora as evidenced from the finding that germ-free and phytoestrogen-free fed animals do not excrete equol when fed soy, and that equol is not found in the plasma and urine of human newborn or 4-month old infants fed exclusively soy foods from birth due to the fact that the intestinal flora has not yet developed in neonates. (See Setchell et al, The Lancet 1997; 350:23-27.).

The phenolic ring structures of isoflavones enable these compounds to bind estrogen receptors (ER) and mimic estrogen. Although genistein and daidzein bind to ER, it is with a lower affinity when compared to estradiol, and with a greater affinity for ER$\beta$ than to ER$\alpha$. Thus isoflavones, like genistein and the metabolite S-equol, act like natural selective estrogen receptor modulators (SERMs) at various tissue sites throughout the body. In some tissues, there is evidence that phytoestrogens act as estrogen agonists, whereas in others, they display antagonistic characteristics comparable to that of tamoxifen or raloxifene where SERM activity appears to be sex-hormone- and gender-dependent.

While the bulk of the scientific literature has focused on the natural isoflavones in soy or clover, little has been reported on the actions or effects of their intestinally derived metabolites. Equol (7-hydroxy-3(4'hydroxyphenyl)-chroman) represents the major metabolite of the phytoestrogen daidzin, one of the main isoflavones found abundantly in soybeans and soy-foods. Equol, however, is not a phytoestrogen, because it is not a natural constituent of plants. Equol does not occur naturally in any plant-based products. Rather, it is a non-steroidal isoflavone that is exclusively a product of intestinal bacterial metabolism, however, only about 30-40% of humans have the microflora necessary to convert soy isoflavones to equol.

Previous research with equol has identified that equol possesses some weak estrogenic properties, binds sex hormone binding globulin and $\alpha$-fetoprotein, and has antioxidant activity. The S-enantiomer of equol (S-)-equol) is the exclusive equol form found in the urine and plasma of "equol-producing" mammals consuming soy, and is the only equol enantiomer made by human intestinal bacteria. The R- and S- enantiomers conformationally differ, which subsequently influences their biological activity. For example, only the S-enantiomer of equol binds estrogen receptor (ER) subtypes with sufficient affinity to be relevant to usual circulating equol levels reported in humans.

The prostate gland depends on androgen hormone action for its development and growth, and the development of human benign prostatic hyperplasia (BPH) clearly requires a combination of testicular androgens during the aging process. However, testosterone is not the major androgen responsible for growth of the prostate. The principal prostatic androgen is 5$\alpha$-dihydrotestosterone (5$\alpha$-DHT), as evidenced by current treatments of prostatic cancer, which are directed toward reducing 5$\alpha$-DHT with 5$\alpha$-reductase inhibitors. Although not elevated in human BPH, 5$\alpha$-DHT levels in the prostate remain at a constant with aging, despite a decrease in the plasma testosterone concentration. Testosterone is converted to 5$\alpha$-DHT by 5$\alpha$-reductase in prostatic stromal and basal cells. 5$\alpha$-DHT is primarily responsible for prostate development and the pathogenesis of BPH. Inhibitors of 5α-reductase reduce prostate size by 20% to 30%. This reduction in glandular tissue is achieved by the induction of apoptosis, which is histologically manifested by ductal atrophy. 5α-reductase occurs as 2 isoforms, type 1 and type 2, with the prostate expressing predominantly the type-2 isoform, and the liver and skin expressing primarily the type-1 isoform. Patients have been identified with deficiencies in the type-2 5α-reductase, but not type 1. Gene-targeted knockout mice with the type-2 5α-reductase null-mutation demonstrate a phenotype similar to that seen in men with 5α-reductase deficiency. Type-1 5α-reductase knockout male mice are phenotypically normal with respect to reproductive function. Enzymatic activity for 5α-reductase or immunohistochemical detection has been noted in other genitourinary tissues, such as the epididymis, testes, gubernaculum, and corporal cavernosal tissue.

Quantitatively, women secrete greater amounts of androgen than that of estrogen due to the greater adrenal cortical responsiveness by gender. The major circulating steroids generally classified as androgens include dehydroepiandrosterone sulphate (DHEAS), dehydroepiandrosterone (DHEA) (originating from the adrenal cortex), androstenedione (A), testosterone (T), and 5α-DHT in descending order of serum concentration, though only the latter two bind the androgen receptor to a significant degree. The other three steroids are better considered as pro-androgens. 5α DHT is primarily a peripheral product of testosterone metabolism. Testosterone circulates both in its free form, and bound to protein including albumin and sex steroid hormone-binding globulin (SHBG), the levels of which are an important determinant of free testosterone concentration. The postmenopausal ovary is an androgen-secreting organ and the levels of testosterone are not directly influenced by the menopausal transition or the occurrence of menopause.

The work of some research has focused on the development of steroidal compounds for the treatment of androgen dependent diseases such as: hirsutism, androgenic alopecia, benign prostatic hyperplasia (BPH) and prostate cancer. DHT has been implicated as a causative factor in the progression of these diseases, largely through the clinical evaluation of males who are genetically deficient of steroid 5α-reductase enzyme. As a result of such studies, the inhibition of this enzyme has become a pharmacological strategy for the design and synthesis of new antiandrogenic drugs. However, it is unclear whether inhibition of 5α-reductase will have a deleterious impact on the system, as evidenced by contraindications arising from reported side effects of conventional treatments using 5α-reducatse inhibitors, such as decreased libido, erectile dysfunction and ejaculatory disorders. The development of different strategies that target the inhibition of DHT effects would be a major advance in the therapy of androgen-mediated conditions.

Despite the recent gains in understanding the pharmacology of equol as it pertains to estrogen actions, our research showing potent antiandrogen effects of equol is unique and novel and opens new approaches to preventing or treating androgen-related conditions. Binding or sequestering 5α-DHT would provide a means for inhibiting its effect on 5α-DHT-sensitive tissues. There is no known ligand that is specific for 5α-DHT, but such an agent would have distinct advantages over non-discriminatory compounds that target the androgen receptor directly or the enzymes involved in androgen synthesis.

## BRIEF SUMMARY OF THE INVENTION

The present invention relates to a method of co-mediating androgen hormone action and estrogen hormone action, that ameliorate one or more physiological and pathophysiological conditions/disorders of the skin in human and non-human species, by administering an enantiomeric equol comprising S-equol, in an amount sufficient to bind free 5α-dihydrotestosterone, thereby inhibiting its binding with androgen receptors, and to bind estrogen receptor subtypes.

The present invention also relates to a method of mediating androgen hormones action that ameliorates one or more physiological and pathophysiological conditions/disorders of the skin in human and non-human species, by administering an enantiomeric equol comprising R-equol, in an amount sufficient to bind free 5α-dihydrotestosterone and inhibit its binding with androgen receptors.

The present invention further relates to a method of treating and preventing androgen-related diseases mediated by androgen hormone action, by administering an enantiomeric equol comprising S-equol, in an amount sufficient to bind free 5α-dihydrotestosterone, thereby inhibiting its binding with androgen receptors, and to bind estrogen receptor subtypes.

The present invention can also relate to a method of treating and preventing androgen-related diseases mediated by androgen hormone action, by administering an enantiomeric equol comprising R-equol, in an amount sufficient to bind free 5α-dihydrotestosterone and inhibit its binding with androgen receptors.

The present invention also relates to a use of an enantiomer of equol comprising S-equol, for treating and preventing androgen-related diseases mediated by androgen hormone action, by administering an enantiomeric equol comprising S-equol, in an amount sufficient to bind free 5α-dihydrotestosterone and inhibit its binding with androgen receptors, and to bind estrogen receptor subtypes.

The present invention also relates to a method of providing a personalized treatment of one or more physiological and pathophysiological conditions/disorders of the skin in human and non-human species, mediated both by DHT and the estrogen receptors, comprising: 1) assessing the one or more disease states or conditions of a patient; 2) assessing the equol-producer status of the patient; 3) determining an optimally beneficial course of treatment, selected from the group consisting of a) a mode of administration, b) a dose amount, c) a dose interval, and d) the enantiomeric ratio of the equol dose.

The methods and compositions of the present invention are useful in the treatment and amelioration of a variety of skin condition/disorders selected from the group consisting of: skin integrity, collagen production, elastin production, elastase, skin thickness, blood flow in the skin, skin turgor, skin moisture content, vaginal dryness, prevention of collagen and elastin, breakdown by matrix metalloproteinases, repair and prevention of wrinkles in skin, enhancing glycoaminoglycans and hyaluronic acid for improved skin appearance, wound healing, improvement of scars in skin, decrease oily skin by improving sebaceous gland function, skin age spots and, acne, male and female pattern baldness, hirsutism, scalp, facial and body hair health and growth, apocrine (sweat) gland function, inflammation of the skin, immune function in the skin, skin pore size, skin temperature and skin and hair abnormalities in steroid hormone synthesis/hormone action, metabolism of steroids and binding steroid receptors involving androgenic and/or estrogenic effects.

## BRIEF DESCRIPTION OF THE DRAWINGS

FIGURE 1 shows the chemical structures of S-equol and R-equol enantiomers

FIGURE 2 shows an appearance/disappearance plot of R-equol in plasma after oral administration of R-equol to a healthy adult.

FIGURE 3 shows a distinct peak in [$^3$H] 5$\alpha$-DHT + equol but not [$^3$H] 5$\alpha$-DHT alone.

FIGURE 4A shows two distinct peaks in [$^3$H] 5$\alpha$-DHT + equol incubated with prostate (A).

FIGURE 4B shows only a single peak is present in [$^3$H] 5$\alpha$-DHT incubated with prostate (B).

FIGURE 5 shows the specific binding of equol to [$^3$H] 5$\alpha$-DHT.

FIGURE 6 shows serum glucose levels from male rats (non-fasting) fed either a Phyto-600 or Phyto-Free diet.

FIGURE 7 shows thyroid (T3) serum levels in male rats fed either a Phyto-600 or Phyto-Free diet.

FIGURE 8 shows testes weight from three groups of rats on a Phyto-Free diet 28 days after receiving equol or vehicle injections.

FIGURE 9A shows the distribution of estrogen receptor beta (ER-$\beta$), 5$\alpha$-reductase enzyme (5$\alpha$-R) and androgen receptors (AR) in non-balding skin.

FIGURE 9B shows the distribution of ER-$\beta$, 5$\alpha$-R and AR in hair follicle bulb of human skin.

FIGURE 9C shows the distribution of ER-$\beta$, 5$\alpha$-R and AR in sebaceous gland of human skin. !

FIGURE 10 shows procollagen synthesis in epidermis following incubation with control substances or equol (racemic mixture) added to tissue culture media.

FIGURE 11 shows averaged procollagen synthesis in epidermis plus dermis following incubation with control substances or equol (racemic mixture) added to tissue culture media.

FIGURE 12 shows procollagen synthesis in derimis following incubation with control substances or equol (racemic mixture) added to tissue culture media.

FIGURE 13 shows shows metabolic activity, as measured by MTT Assay following incubation of human dermal monolayer fibroblasts with 0.01%, 0.001%, and 0.0001% equol, 0.01%, 0.001%, and 0.0001% 17$\beta$-estradiol, vehicle, or ascorbate added to the culture media.

FIGURE 14 shows collagen deposition by, as measured by Collagen Type I C-Terminal Propeptide ELISA, following incubation of human dermal monolayer fibroblasts with 0.01%, 0.001%, and 0.0001% equol, 0.01%, 0.001%, and 0.0001% 17$\beta$-estradiol, vehicle, or ascorbate added to the culture media.

FIGURE 15 shows metabolic activity, as measures by MTT Assay following incubation of human dermal monolayer fibroblasts with vehicle or 0.001% equol added to the culture media. Horizontal line indicates baseline as determined by untreated control cultures.

FIGURE 16 collagen deposition by, as measured by Collagen Type I C-Terminal Propeptide ELISA, following incubation of human dermal monolayer fibroblasts with transcutol vehicle, 0.0001% equol, or ascorbate added to tissue culture media. Horizontal line indicates baseline as determined by untreated control cultures.

FIGURE 17 shows metabolic activity, as measured by MTT Assay following incubation of human dermal monolayer fibroblasts with untreated media, ascorbate, 0.001% equol, 0.001% 5$\alpha$-DHT, or a combination of 0.001% equol and 0.001% 5$\alpha$-DHT added to tissue culture media. Horizontal dashed line indicates baseline as determined by untreated control cultures.

FIGURE 18 shows prostate-specific antigen (PSA) levels secreted by prostate cancer cells following the incubation with vehicle, 10, 1, or 0.1 nM 5$\alpha$-DHT, 100, 10, or 1 nM equol, or combinations of 5$\alpha$-DHT and equol added to tissue culture media.

FIGURE 19 shows fluorescence activated cell sorter (FACS) analysis of collagen type I protein expression in 3-dimensional (3-D) cultures of human dermal monolayer fibroblast following incubation with vehicle, 10 nM equol or 10 nM 17$\beta$-estradiol added to tissue culture media.

FIGURE 20 shows FACS analysis of collagen type III protein expression in 3-D cultures of human dermal monolayer fibroblast following incubation with vehicle, 10 nM equol or 10 nM 17β-estradiol added to tissue culture media.
FIGURE 21 shows FACS analysis of matrix metalloproteinase-3 (MMP-3) protein expression in 3-D cultures of human dermal monolayer fibroblast following incubation with vehicle, 10 equol or 10 nM 17β-estradiol added to tissue culture media
FIGURE 22 shows FACS analysis of elastin protein expression in 3-D cultures of human dermal monolayer fibroblast following incubation with vehicle, 10 nM equol or 10 nM 17β-estradiol added to tissue culture media.
FIGURE 23 shows FACS analysis of elastase protein expression in 3-D cultures of human dermal monolayer fibroblast following incubation with vehicle, 10 nM equol or 10 nM 17β-estradiol added to tissue culture media.
FIGURE 24 shows cell cycle analysis of apoptosis by FACS in 3-D cultures of human dermal monolayer fibroblast following incubation with vehicle, 10 nM equol or 10 nM 17β-estradiol added to tissue culture media.
FIGURE 25 shows cell cycle analysis of cell cycling in S-G2M phases by FACS in 3-D cultures of human dermal monolayer fibroblast following incubation with vehicle, 10 nM equol or 10 nM 17β-estradiol added to tissue culture media.
FIGURE 26 shows the skin tail temperature of male rats after receiving vehicle or equol injections for 25 consecutive days.
FIGURE 27 shows the results of human dermal monolayer fibroblast collagen deposition measured by Collagen Type I C-Terminal Propeptide ELISA following incubation with vehicle, 10 nM equol, 10 nM 17β-estradiol, or ascorbate added to tissue culture media. β-estradiol, or ascorbate added to
FIGURE 28 shows the ocular irritection model for testing irritant characteristics of vehicle (ethanol), equol-racemic or s-equol.
FIGURE 29 shows the dermal irritection model for testing irritant characteristics of vehicle (ethanol), equol-racemic or s-equol.

## DETAILED DESCRIPTION OF THE INVENTION

As used herein, the term "skin" refers to cell layers comprising the integument of a human or non-human individual, and its structural components such as hair, hair follicles, sebaceous glands, apocrine (sweat) glands, fingernails and toenails. Furthermore, the term "skin" as used herein encompasses tissues of the mucous membranes extending from the adjoining skin, such as the mouth and oral cavity, nose and nasal passages, eyes and eyelids, ears and outer ear canals, and the perineum and tissues of the anal and urogenital orifices
As used herein, the term "affected area" refers to a region of the skin that is to be treated with a therapeutic molecule or compound containing a therapeutic molecule. The affected area may be the site of a skin condition or disease for which treatment is sought. In some cases, the affected area may encompass all skin on an individual. Alternatively, the affected area may be a site for which improvement of a cosmetic nature is sought, and can also include all skin on an individual.
As used herein, the term "systemic" or "systemically" refers to a mode of administration of a therapy that reaches an affected area of skin via the blood stream or lymphatic system. Examples of a systemic treatment include, but are not limited to, oral gavage or ingestion, intravenous or subdermal pump infusion, and injection via intramuscular, intraperitoneal, hypodermic or subdermic injection.
As used herein, the term "topical" or "topically" refers to a mode of administration that is applied directly to an affected area of the skin. Examples of a topical treatment include, but are not limited to application of cream, lotion, shampoo, conditioning lotion, spray, a pad, a bandage, a diaper, a proistened towelette, or transdermal patch; and local administration via intracutaneous injection or introduction of a lozenge or suppository.
As used herein, the term "skin parameters" refers to a variety of indicators of skin health, including but not limited to levels qf collagen and elastin production, elastase, skin thickness, blood flow in the skin, skin turgor and moisture content, prevention of collagen and elastin breakdown by matrix metalloproteinases, absence of wrinkles in skin, presence of glycoaminoglycans and hyaluronic acid for normal skin appearance, ability of skin wounds to heal, normal sebaceous gland function, absence of skin age spots or pigmentation dysfunction, skin pore size, skin temperature, and normal growth of hair and nails.
As used herein, the term "skin integrity" refers to the presence of collagen and elastin in the extracellular matrix that gives skin its ability to stretch and retract to allow movement.
Equol (7-hydroxy-3(4'hydroxyphenyl)-chroman) represents the major metabolite of daidzin and daidzein, isoflavones found abundantly in soybeans and soy-foods, and is an important biologically active molecule. In animals, such as rodents, fed a phytoestrogen-rich diet, the major circulating isoflavone is equol, which accounts for 70-90% of the total circulating isoflavone levels. However, this is not the case in humans.
Equol is formed following the hydrolysis of the glycoside conjugates of daidzin from soy, and the methoxylated isoflavone formononetin, or its glycosidic conjugates found in clover. Once formed, equol appears to be metabolically inert, undergoing no further biotransformation, save phase II metabolism or a minor degree of additional hydroxylation in the liver. As with daidzein and genistein, the predominant phase II reactions are glucuronidation and, to a lesser extent, sulfation. Following the original discovery that equol's presence in urine was a function of soy food ingestion, it was observed that approximately 50 - 70% of the adult human population did not excrete equol in urine even when challenged daily with soy foods, for reasons that are unclear. Furthermore, even when the pure isoflavone compounds are administered, thereby removing any influence of the food matrix, it has been shown that many people do not convert daidzein to equol. This phenomenon has led to the terminology of a person being an 'equol-producer' or 'non-equol producer' (or 'poor equol-producer') to describe these two distinct populations.

Cut-off values have been empirically derived permitting assignment of individuals to either of these categories. People who have plasma equol concentrations of less than 10 ng/mL (40 nmol/L) can be classified as 'non-equol producers' and where levels are above 10 ng/mL (40 nmol/L) this defines 'equol producers'. This distinction can also be derived from the levels in urine, an equol producer being someone excreting greater than 1000 nmol/L. Although the excretion of equol is highly variable among individuals there is a large demarcation between those that can produce equol and those that cannot, consistent with a precursor-product relationship in enzyme kinetics catalyzing the reaction. There is consequently an inverse relationship between urinary daidzein and equol levels, and thus far no significant gender differences have been defined.

A mechanism of action for equol has been identified with important ramifications in skin health and disease and which indicates a broad and important usage for equol in the treatment of androgen and/or estrogen mediated pathologies of skin and hair Equol can act as an anti-androgen or estrogen receptor agonist or antagonist. The anti-androgenic properties of equol are unique in that equol does not bind the androgen receptor (AR) but rather, specifically binds 5α-dihydrotestosterone (5α-DHT) with high affinity, thereby preventing 5α-DHT from binding the AR. Furthermore, both the R- and S-enantiomers of equol specifically bind 5α-DHT, sequester 5α-DHT from the AR and block 5α-DHT's actions in physiological processes in vivo. Racemic equol, which constitutes R-equol and S-equol, and R-equol, or S-equol alone, selectively bind 5α-DHT.

In mammals, there are two principal androgens, testosterone and its 5α-reduced metabolite, 5α-DHT. 5α-DHT is recognized as the most potent androgen in the mammalian body. The AR, which is encoded by a single-copy gene located on the human X-chromosome, specifically mediates the actions of androgens. Although both testosterone and 5α-DHT bind the AR, certain tissues (i.e. prostate gland, hair follicles, etc.) that are only slightly influenced by testosterone are greatly influenced by 5α-DHT. Furthermore, 5α-DHT has been implicated in a number of diseases and disorders. Because equol specifically binds and prevents the actions of 5α-DHT, there is an indication for a broad and important usage for equol in the treatment of androgen-mediated pathologies of skin and hair.

Equol has a structure similar to the steroidal estrogen estradiol. FIGURE 1 shows the chemical structures of R-equol and S-equol. Equol is unique among the isoflavones in that it possesses a chiral center and as such exists as two distinct enantiomeric forms, the R- and S- enantiomers. The R- and S- enantiomers conformationally differ and this is predicted to influence how an equol enantiomer fits into the binding site in the cavity of the dimerized ER complex, and how it binds with 5α-DHT.

Approximately 50% of equol circulates in the free or unbound form in humans, and this is considerable greater than the proportion of free daidzein (18.7%) or estradiol (4.6%) in plasma. Since it is the unbound fraction that is available for receptor occupancy, and presumably for binding 5α-DHT, this would effectively contribute to enhancing the overall potency of equol.

All known previous studies on equol appear to have been conducted with the racemic form of equol. There has in general been a lack of appreciation that two forms of equol exist or that the enantiomers may behave differently, and to our knowledge no previous study has reported on the specific actions or activity of the individual enantiomers. R- and S-equol specifically bind 5α-dihydrotestone (5α-DHT). Equol racemic, R-equol or S-equol, does not bind the androgen receptor (AR). Compared to 17β-estradiol the relative binding affinities of the R- and S-equol enantiomer for ERα 1/210 and 1/49 less than that of 17β-estradiol, respectively. However, the S-equol enantiomer seems to be largely ERβ-selective with a relatively high affinity for ERβ. Enantiomer S-equol binds ERβ at similar concentrations to that of 17β-estradiol [equol, Kd = 0.7 nM vs. 17β-estradiol, Kd = 0.15 nm], but its preferential affinity for ERβ subtype defines S-equol as a SERM. The R-equol enantiomer binds at approximately 1/100 the affinity, however, if R-equol is present in extremely high concentrations, it does have SERM properties. Thus, S- and R-equol have the capability to selectively bind the most potent circulating androgen, 5α-DHT, and S-equol has sufficient affinity for ERβ to classify it as having SERM properties.

The ability of both S-equol, the natural metabolite of daidzein, and R-equol to antagonize the actions of the potent androgen-dihydrotestosterone, 5α-DHT opens up opportunities for cosmetic, dietary, nutraceutical, and pharmacological approaches to prevention and treatment of disease where the potent androgen 5α-DHT plays a detrimental role, including, but not restricted to, prostate cancer, obesity, skin diseases, and hair loss. Additionally, the estrogenic actions of S-equol can also be of benefit in treating or preventing BPH and prostate cancer because the combined actions of equol acting at the estrogen receptor level and as an antiandrogen.

R-equol, although not naturally occurring, is of considerable importance because of its ability to modulate androgen-mediated processes in the body. In binding studies, equol enantiomers specifically bind 5α-dihydrotestosterone (5α-DHT), but not testosterone, DHEA or estrogen. By doing so, equol sequesters 5α-DHT from the androgen receptor without directly binding the androgen receptor itself. *In vivo* studies demonstrate that equol treatment of intact male rats significantly decreased prostate and epididymis but not testes weights. In castrated male rats treated with 5α-DHT after administering equol, equol blocked 5α-DHT's trophic effects on the prostate gland and its negative feedback effects on plasma luteinizing hormone (LH) levels.

Equol can act as an anti-androgen, by specifically binding 5α-DHT and preventing 5α-DHT from binding to the androgen receptor (AR) without itself binding the AR. Further 5α-DHT that has already been bound to the AR will not be competitively bound by enantiomeric equol. The enantiomeric equol may be brought into contact with the 5α-DHT *in vitro* or *in vivo.* When the 5α-DHT is to be contacted *is vivo*, the equol, may be administered by any route that allows absorption of equol to the blood stream or into the skin when applied topically. Biologically available 5α-DHT is free and unbound by any native ligand prior to binding with equal

Reproductive organs such as the prostate and epididymis are known to be under androgenic control Previous data has shown that before puberty, when circulating androgen levels are very low, rats fed a diet containing high levels of soy-derived isoflavone have prostate weights that are not altered by the consumption of this diet. However, after puberty when androgen levels increase, prostate weights are significantly decreased in phytoestrogen-rich-diet fed rats compared to animals fed a phytoestrogen-free diet. These data are similar to the present finding that equol-treated intact rats display significant decreases in prostate and epididymis weights, without alterations in testes or pituitary weights. Notably, if the prostate and epididymal values are standardized to body weight (per 100 grams) the ratios are still significantly different between equol-treated and control values. Equol also blocked 5α-DHT's androgenic trophic influence on the prostate and epididymis, without significantly altering testosterone levels.

5α-DHT has negative feedback effects on circulating plasma levels of luteinizing hormone (LH). Equol significantly increases LH levels by binding 5α-DHT and preventing this feedback effect. Equol completely reverses the inhibitory action of 5α-DHT on LH levels in gonadectomized (GDX) males, whereas 5α-DHT plus equol-treated male rats display LH levels similar to that of control values. These data further suggest that equol has the specific ability to bind 5α-DHT, presumably in the blood circulation system, and block the hormonal action of 5α-DHT in suppressing LH production or secretion. Therefore an embodiment of the present invention is a method of modulating LH levels in an individual by contacting the 5α-DHT of the individual with enantiomeric equol. The equol can be administered by any route that allows absorption of equol into the skin or blood stream, with the amount administered in accordance with the nature of the ailment to be treated and size of the individual. In some cases, it may be desirable to provide a combination of both systemic and topical treatments.

Enantiomeric equol can be prepared by chemical synthesis, and can be isolated from racemic mixtures, typically using a chiral-phase column, by known means. S-equol can be made with high enantioselectivity using a biological process that employs the equol-producing microorganism associated with metabolism of equol from isoflavones such as daidzein. These means are described in PCT Patent Publication WO04-009035, incorporated herein by reference.

Treatment of Disease by Administering S-Equol, R-equol, and Mixtures:

This present invention provides a means for an individual subject to overcome the problem of not being able to produce equol *in vivo,* or to supply R-equol in particular, by providing delivery of equol enantiomers, the S-equol or R-equol, racemic or non-racemic mixtures of S-equol and R-equol directly, circumventing the need for intestinal bacteria for its production or for the need to consume soy foods with equol's precursor isoflavones. The delivery of S-equol can also supplement the *in vivo* production of S-equol in 'equol-producers', as well as in 'non-equol producers'.

Supplementing the diet of an equol producer with an equol enantiomer or mixture, can provide benefits when the ordinary level of S-equol produced by the equol producer is inadequate because of 1) insufficient consumption of isoflavones to produce equol, 2) antibiotic use that ablates the activity of intestinal bacteria to make equol from precursor isoflavones, or 3) other health factors that impact the level of equol production or absorption, such as short bowel syndrome or surgical construction of an intestinal stoma such as ileostomy. In addition, a supplemental level of equol is believed to provide enhanced effect on the health and well-being of the person.

This invention provides a method for delivering S-equol, R-equol, racemic equol, or non-racemic mixtures of equol, in sufficient amounts to have health benefits toward androgen-related diseases and conditions associated therewith. The anti-androgenic activity of equol can affect a number of tissues throughout the body. In particular, the blocking of androgenic activity of 5α-DHT can be beneficial for the treatment and prevention of: female- and male-pattern baldness, facial and body hair growth (hirsutism), skin health (acne, anti-aging such as wrinkle prevention and repair and anti-photo aging), and skin integrity (collagen and elastin robustness). The method can be a topical administration, a systemic administration, or a combination of topical and systemic administration.

For topical administration, the concentration of equol applied to an affected area of skin will from 0.01% to 10%. Typically, 0.01% to is effective to induce increased skin integrity, collagen production, elastin production, elastase, skin thickness, blood flow in the skin, skin turgor, skin moisture content, prevention of collagen and elastin breakdown by matrix metalloproteinases, repair and prevention of wrinkles in skin, enhancing glycoaminoglycans and hyaluronic acid for improved skin appearance, wound healing, improvement of scars in skin, decrease oily skin by improving sebaceous gland function, skin age spots and skin lightening, acne, male and female pattern baldness, hirsutism, scalp, facial and body hair health and growth, apocrine (sweat) gland function, inflammation of the skin, immune function in the skin, skin pore size, skin temperature and skin and hair abnormalities in steroid hormone synthesis/hormone action, metabolism of steroids and binding steroid receptors involving androgenic and/or estrogenic effects. In some cases, a higher dose is required due to the presence of a skin condition or disease, or because the patient is a non-equol producer. In this situation, the concentration of topically-applied equol can be up to 10%, topical administration may be performed more frequently, or systemic administration may be used in combination with or in place of topical administration.

For systemic administration, the amount of composition comprising the equol is administered in an amount sufficient to produce a transient level of enantiomeric equol in the blood plasma of the mammal of at least 5 nanograms per milliliter (ng/mL), more typically at least 10 ng/mL or greater, or transient levels of enantiomeric equol in urine of greater than 1000 nmol/L. Typically, the composition is administered orally in a dose amount of at least about 1 mg, more typically of at least 5 mg, and of up to 200 mg, more typically, up to 50 mg, of enantiomeric equol. Typical plasma concentrations of R-equol in plasma after oral administration of 20 mg of R-equol enantiomer to a healthy adult is shown by the pharmacokinetics of the plasma appearance/disappearance plots of R-equol in FIGURE 2. A typical level of bioavailability of S-equol in plasma after oral administration of 20 mg of S-equol to a healthy adult is similar to that shown for R-equol.

The ability to deliver R- and/or S-equol in sufficient amounts is believed to provide several advantages over delivery of a racemic mixture of equol. First, the potency of R-equol or S-equol alone would typically be at least twice that of the racemic mixture. Second, the human body only produces S-equol, and therefore, a composition comprising only S-equol represents a "natural" product with an ingredient, S-equol, with which the body is familiar. Third, since the R-equol enantiomer has unique properties, a treatment composition comprising only, or substantially only, the R-enantiomer can produce beneficial and/or therapeutic effects. And fourth, administration of R-equol would supplement any endogenous S-equol present and allow for both estrogenic and anti-androgenic actions to occur in the body.

The invention includes the use of enantiomeric equol to treat and prevent diseases and conditions related to male- and female-pattern baldness. 5α-DHT is a known cause of scalp hair loss. An androgen, specifically the principal circulating androgen, testosterone, is converted to the more potent androgen, dihydrotestosterone (5α-DHT) (in the hair follicle), and the hormonal action of 5α-DHT on scalp hair follicles cause hair loss. Thus, if the hormonal action of 5α-DHT can be blocked, such as by the use in the present invention of equol to bind 5α-DHT in the circulation (within blood vessels) and within the hair follicle], then scalp hair loss can be decreased or prevented.

The invention includes the use of enantiomeric equol to treat and prevent diseases and conditions related facial and body hair. Facial and body hair are regulated by androgens, but oppositely to that of the regulation of scalp hair. Specifically, the more potent androgen, 5α-DHT, increases facial and body hair. 5α-DHT also increases the production of sebum (oil) from the sebaceous gland, which can contribute to an increase in acne. Thus, the binding of 5α-DHT by equol can cause a decrease in facial and body hair and in secretion of sebum (oil), and a reduction or prevention of acne.

The invention includes the use of enantiomeric equol to treat and prevent diseases and conditions related to skin effects, skin quality and integrity, skin aging, skin photo-aging, and skin pigmentation and lightening. Estrogens, before but especially after menopause, improve skin health by increasing elastin and collagen content to improve skin characteristics or robustness. Also, when skin is damaged by acne or other skin disruptions (scratches, popping pimples or minor cuts, etc.), the repair mechanism is faster and the skin heals better if estrogen or estrogen-like compounds, such as equol, are present. It is believed that an enantiomeric equol, and particularly S-equol or a mixture of the enantiomers or racemic equol stimulates elastin and collagen, and also can protect against photo-aging. The ability of equol to block the hormone action of 5α-DHT can decrease sebum oil production from the sebaceous gland, which can decrease or eliminate acne. Since S-equol binds estrogen receptor(s) (mainly ERβ), the protective effects of this estrogen-like molecule stimulates elastin and collagen in the skin. Additionally, since equol is a strong antioxidant, it can protect the skin from aging, including photo-aging.

Sex steroid hormones are involved in the regulation of skin development and functions, such as secretions, as well as in some skin pathological disorders. It is well established that the actions of estrogens and androgens hormone are mediated by the presence of their receptors in skin, hair and glands associated with skin. See Pelletier and Ren, Histol Histopathology, 19: 629-636, 2004. For example, androgen receptors (AR) have been localized in most keratinocytes in the epidermis/dermis and AR was seen in approximately 10% of fibroblasts. However, in sebaceous glands, AR is abundant in basal cells and sebocytes. In hair follicles AR expression is restricted to dermal papillar cells. ERβ is highly expressed in the epidermis, sebaceous glands (basal cells and sebocytes) and eccrine sweat glands. In hair follicles, ERβ is widely expressed in dermal papilla cells, inner sheath cells, matrix cells and outer sheath cells including the bulge region.

Since equol is a metabolite of daidzein and possesses the characteristics of selective estrogen receptor modulators (SERMS), where in some cells and tissue sites it acts like an estrogen agonist and in others an estrogen antagonist, it is reasonable to propose that equol can have dual estrogen-like hormone actions at various cells/tissue containing ERβ. It has been established that equol (racemic) has the ability to bind ERβ through its enantiomer S-equol, since R-equol has low affinity for ERa or ERβ. However, both S-equol and R-equol are of considerable importance because of their ability to specifically bind and biologically inactive 5α-DHT that plays a major role in: a) scalp and facial/body hair follicle growth, such as androgenetic alopecia or male-pattern baldness, and hirsutism in women or female-pattern baldness, b) acne and sebaceous gland function, c) wound healing and d) skin disorders such as apocrine gland dysfunction, hidradentitis suppurativa or osmidrosis. Finally, estrogens are known to positively influenced skin parameters, wound healing, hair follicle health, sebaceous and apocrine gland function, epidermal and hair follicle pigmentation, and malignant melanoma.

Estrogen is known to be a major hormonal factor in the maintenance of human skin. It is known to stimulate collagen production in the dermis, increase skin thickness, increase the vascularization of the skin and increase the mitotic activity of the epidermis. See Brincat MP, Maturitas, 29:107-117, 2000; Punnonen R, Acta Obstet Gynecol Scand Suppl, 21: 3-44, 1972; Hasselquist, MB et al., J Clin Endocrinol Metab, 50: 76-82, 1980; and Shah MG and Maibach HI, Am J Clin Dermatol, 2:143-150, 2001. Specifically, estrogen is known to be a natural modulator of matrix metalloproteinases (MMP), as described by Pirila E et al., Curr Med Chem, 8:281-294, 2001. MMPs are known to break down collagen and elastin. This could be due to environmental (exposure to chemicals, pollution, extreme temperature environments - cold or heat), mechanical (contractions of facial muscles, such as smiling, frowning, smoking or drinking from a straw) or biological aging. These factors are influenced by genetics and the natural processes of aging, wherein the skin becomes thin, wrinkles appear due to the reduction in collagen and especially elastin, and the robust appearance of the skin declines. Estrogen treatment has also been shown to increase the concentrations of glycoaminoglycans (acid mucopolysaccharides and hyaluronic acid) that enhance the water content or moisture of skin and influence skin turgor. See also Raine-Fenning NJ et al., Am J Clin Dermatol, 4:371-378,2003.

The invention includes the use of S-equol, or racemic or non-racemic mixtures of S- and R-equol to ameliorate or block the negative effects of cancer therapies that involve estrogen receptors, such as tamoxifen. Tamoxifen treatment has been shown to cause vaginal dryness, which can be ameliorated by equol through its SERM actions. The invention can be used in a similar manner to ameliorate vaginal dryness that accompanies menopause or post-menopause vaginal dryness.

The invention includes the use of S-equol, or racemic or non-racemic mixtures of S- and R-equol to block the negative effects of 5α-DHT and decrease MMPs to positively influence skin collagen, elastin, vascularization and skin thickness and skin turgor and slow down the process of environmental, mechanical and biological aging.

Estrogen is important in the rate and quality of wound healing. See Pirila Eet al., Curr Med Chem, 8:281-294, 2001 and Ashcroft GE et aL, Nat Med, 3:1209-1215, 1997. It has been demonstrated that ERβ is the predominant estrogen receptor in adult human scalp skin and the pilosebaceous unit (hair follicle) of the skin. See Thornton MJ et al., Exp Dermatol, 12: 181-190, 2003 and Thornton MJ, et al., J Invest Dermatol Symp Proc, 8: 100-103, 2003. Furthermore, it has been reported that physiological levels of 5α-DHT depress wound healing by impairing immune function and promoting inflammation. See Nitsch SM et al., Arch Surg, 139:157-163, 2004 and Gilliver SC et al., Thromb Haemost, 90: 978-985, 2003. MMPs are also involved in wound healing that are modulated by estrogens.

The invention includes the use of S-equol, or racemic or non-racemic mixtures of R- and S-equol, via its R- and S- enantiomers binding to extracellular and intracelluar 5α-DHT, to biologically inactivate this potent androgen and positively influence wound healing. At the same time a racemic or non-racemic mixture of Rand S-equol, will improve the rate and quality of wound healing via an ERβ hormone-mediated mechanism.

A built-in rhythm of activity in scalp hair follicles results in the growth of new hairs and the molting of old ones. This activity is known to be under the influence of steroid hormones. Scalp hair follicle growth is specifically inhibited by 5α-DHT, while at the same time, facial and body hair follicles are stimulated by *5α-DHT.* It is known that postmenopausal women experience female-pattern balding patterns due to the loss of ovarian steroid hormones and the increased ratio of androgens/estrogens during, this period, as described by Brincat (see reference above). Postmenopausal women also experience hirsutism, or increased facial and body hair growth, during this period, and with increased androgen production during the pre-menopausal period. Pre-menopausal women also experience hirstutism with increased androgen production. See Reed MJ and Franks S, Baillieres Clin Obstet Gynaecol, 2: 581-595, 1988. Castrated males, who have low levels of androgens, or humans with genetic 5a-DHT-reductase deficiency do not experience male-pattern baldness (Trueb RM, Exp Gerontol, 37: 981-990, 2002.).

It is also known from in *vitro* and *in vivo* studies that estrogens increase the hair follicle growth or life cycle and stimulate the secretion of vascular endothelial growth factor (VEGF) in dermal papilla cells that influence blood flow to the hair follicle. See also Lachgar S et al., J Invest Dermatol Symp Proc, 4: 290-295, 1999.

The invention includes the use of S-equol, or racemic or non-racemic mixtures of R- and S-equol_to biologically inactivate the potent androgen 5α-DHT and inhibit the negative effects on scalp hair follicle growth in both men and women. Equol will stimulate hair follicle life cycle and enhance vascular endothelial growth factor (VEGF) in dermal papilla cells to positively influence scalp hair growth. Conversely, R-equol, and/or S-equol can block the hormonal actions of 5α-DHT, and facial and body hair growth will be reduced.

Estrogens decrease the size and inhibit sebaceous gland secretion in males and females. See Pochi PE and Strauss JS, J Invest Dermatol, 62: 191-210, 1974 and Larie F et al., Horm Res, 54: 218-229, 2000. ERβ is widely and highly expressed in the sebaceous gland and estrogen hormone action via this receptor apparently reduces oil gland secretion associated with hair follicles. On the other hand, androgen receptors in the sebaceous glands are activated by 5α-DHT. 5α-DHT in the sebaceous gland stimulates oil production that is associated with attracting bacteria and thus the promotion and production of acne.

The invention includes the use of S-equol, or racemic or non-racemic mixtures of R- and S-equol to biologically inactivate the potent androgen 5α-DHT and inhibit the production of oil secretion from the sebaceous gland to reduce the incidence of acne. A combination of enantiomers (R-equol and S-equol) will reduce the size and inhibit the production of oil from the sebaceous gland to assist in the amelioration or prevention of acne.

The apocrine gland develops from the outer root sheath of the hair follicle and remains attached to it. Apocrine glands are associated with hairy regions of the body that produce sweat mostly from the armpits and groin region, as described by Jakubovic HR et al., in Dermatology, Third Edition, Philadelphia, W.B. Saunders, 1992, pp. 69-77. Hidradentitis suppurativa and osmidosis are conditions due to inflammation of the large sweat glands associated with the armpits and groin. See Sato T et al., Br J Dermatol, 139: 806-810, 1998. Patients with these disorders have excessive or abnormal odor derived from apocrine sweat (osmidrosis). The condition is more common in females and appears to improve with estrogen and/or antiandrogen treatments, suggesting that these specialized glands are regulated by estrogens and androgens. See also Offidani A et al., J Clin Pathol, 52: 829-832, 1999. Specifically when androgen hormone action has been examined, high levels of 5α-reductase activity have been detected in the apocrine glands in patients who suffer from excessive or abnormal odor for their apocrine (sweat) glands, and the action of 5α-DHT has been implicated in these conditions.

The invention includes the use of S-equol, or racemic or non-racemic mixtures of R- and S-equol can biologically inactivate the potent androgen hormone 5α-DHT and inhibit the production of sweat secretion from apocrine glands and reduce the incidence of hidradentitis and osmidrosis. Concomitantly, in a combination of the ratios of enantiomers (R-equol and S-equol), equol will reduce production of sweat from aprocrine glands to assist in the prevention of hidradentitis and osmidrosis.

Several studies have shown that epidermal melanocytes are estrogen responsive. There are several reports of estrogen-containing oral contraceptives causing hyperpigmentation of the face in women, as described by Wade TR et al., Obstet Gynecol, 52: 233-242,1978.

The invention includes the use of S-equol, or racemic or non-racemic mixtures of R- and S-equol to biologically inactivate the potent androgen hormone 5α-DHT and enhance the hormonal action of equol at estrogen receptors. Epidermal melanocytes will be inhibited via the SERM action of equol, and the result will be a skin-lightening effect. Thus, equol can be an effective treatment for age or skin spots, especially on the face and hands.

The invention includes the use of S-equol, or racemic or non-racemic mixtures of R-equol and S-equol to biologically inactivate the potent androgen hormone 5α-DHT and enhance the hormonal action of equol at estrogen receptors. Hair follicle melanocytes are stimulated via the SERM action of equol, and the result is an enhancement of hair pigmentation. See Tobin DJ and Bystryn JC, Pigment Cell Res, 9: 304-310, 1996; Thorton MJ, Exp Dermatology, 11: 487-502, 2002; and Ohuchi A et al., in: Third Intercontinental Meeting of Hair Research Societies, Japan, 2001. Thus, equol can be an effective treatment for modulating the tone and color of hair follicles and thus change hair pigmentation.

Due to the association of hair follicles with the formation of malignant melanomas, the SERM characteristics of equol may influence malignant melanomas in a positive manner. See Kanda N and Watanabe S, J Invest Dermatol, 117: 274-283, 2001; Richardson B et al., Br J Cancer, 80: 2025-2033, 1999; and Durvasula R et al., Climacteric, 5: 1970200, 2002. For example, treatment of human metastatic melanoma cell lines incubated with estradiol inhibited the uptake of $^{3}$H-thymidine, which was counteracted by the administration of an antiestrogen. Moreover, estradiol can inhibit the invasion of human melanomas cells through the activation of fibronectin. Finally the mean age of presentation of malignant melanoma in women is the early fifties, a time concomitant with the onset of menopause. Melanoma has traditionally been considered to be an estrogen-receptor positive tumor, however recent evidence now refutes this.

The invention includes the use of S-equol, or racemic or non-racemic mixtures of R-equol or S-equol to biologically inactivate the potent androgen hormone 5α-DHT and enhance the hormonal action of equol at estrogen receptors. Via the SERM action of equol, the formation of malignant melanomas will be inhibited and hair follicle melanocytes will be stabilized resulting in the prevention and treatment of malignant melanomas.

Other embodiments of the present invention include the use of equol as a diagnostic agent in androgen-related skin or hair disorders as well as disorders arising from disturbances in estrogenic/androgenic balance. In these embodiments, equol is administered to an individual to bind 5α-DHT and thereby prevent 5α-DHT binding to androgen receptors. The changes in estrogenic balance are then measured or the change in androgen-binding is assessed to diagnose or further elucidate androgen-related anomalies of skin or hair.

Equol has been found to improve skin health by increasing elastin and collagen content to improve skin characteristics or robustness. The mechanism of this action is believed to block the hormone action of 5α-DHT that would in turn decrease oil production from the sebaceous glands to decrease acne and other skin disorders. Since equol, and particularly S-equol, binds estrogen receptor(s), the protective effects of the estrogen-like molecules stimulate production of elastin and collagen in the skin that is thought to be mediated via ER-β. In addition, the antioxidant properties of equol protect against photo-aging and, in general, the aging of the skin.

Equol can be administered to bind 5α-DHT prior to or along with other therapeutic moieties in order to assess the binding capacity of 5α-DHT with respect to the therapeutic moiety in question. Also, androgen-binding moieties can be administered following administration of equol to assess the efficacy of the androgen-binding moiety to restore androgen activity and balance estrogenic activity in the absence of 5α-DHT binding. Further, equol can be administered in the presence of 5α-DHT-binding moieties in order to displace these naturally occurring or xenobiotic 5α-DHT-binding moieties from 5α-DHT.

Enantiomeric equol can be orally administered by supplying an oral dosage form of equol, which results in effective absorption of equol to the blood stream. Administration of equol may be made by routes other than oral if desired. For example, it is contemplated that rectal or urethral administration may be used to administer equol for the treatment of enlarged prostate or to prevent prostate enlargement. Additionally, it is contemplated that the active ligand binding site of the equol molecule may be isolated and synthesized for administration, which can provide 5α-DHT binding without the full equol molecule. The dose of the equol molecule or fragment thereof having 5α-DHT-binding abilities is dependent upon the route of administration and the condition to be treated. Based on our *in vivo* studies it is apparent that relatively low doses of equol antagonize much higher doses of 5α-DHT, and this may be explained by the marked differences in the binding of equol to serum protein compared with 5α-DHT. The latter circulates mostly bound to proteins, while equol is 50% free. Generally, a dose sufficient to produce a concentration of equol or active fragments thereof in the bloodstream of the recipient of at least about 0.2 mg equol per kg weight of the recipient and preferably at least about 0.5mg/kg. The dose may be increased dramatically without incurring significant dose-limiting side effects to greater than about 10mg/kg. Oral administration can be effected in microencapsulated forms that can provide delayed or sustained release of the medicament.

Equol can be administered topically, transdermally, and subdermally in a variety of forms, including lotions, ointments, foams (including shaving creams), and sprays, or as an active ingredient on a substrate suitable for topical application, such as a pad, a surgical bandage, an adhesive bandage, a premoistened towellette, an infant or adult incontinent diaper (such as described in US Patent 5,525,346, incorporated herein by reference), a feminine sanitary product, or a transdermal skin patch (such as described in US Patent 5,613,958 and 6,071,531,incorporated herein by reference), electromechanical devices, including micropumps systems (such as described in US Patent 5,693,018 and US Patent 5,848,991, incorporated herein by reference), and subdermal implants (such as described in US Patent 5,468,501, incorporated herein by reference).

A composition useful in the practice of the present invention comprises an at least physiological acceptable quantity of equol that is able to at least partially bind and sequester free 5α-DHT (but not testosterone or DHEA) thereby preventing it binding to the androgen receptor following administration to an individual thereby having important ramifications in health and disease and a broad and important use in the treatment of androgen-mediated pathologies.

A composition containing S-equol, R-equol, a racemic equol mixture, or a non-racemic equol mixture, can be made for oral consumption. The composition or a product containing the composition can be a marketed or institutional food product, a pharmaceutical, and an OTC medicament. A food composition can comprise at least 1 mg, and typically up to 200 mg, enantiomeric equol or equol mixtures, per serving. An orally-administered medicament can comprise at least 1 mg, and typically up to 200 mg, enantiomeric equol or equol mixture, per dose. A product for topical application can comprise at least 0.01%, and up to 10%, by weight S-equol, or R-equol, or enantiomeric mixtures. Selected concentration ranges include from about 0.01% to about 3%, from about 0.1% to about 1%, from about 0.1% to about 3%, from about 0.1% to about 5%, from about 0.3% to about 1%, from about 0.3% to about 3%, from about 0.3% to about 5%, from about 0.5% to about 1%, from about 0.5% to about 3%, and from about 0.5% to about 5%. Typically, 0.01% to 1% is an effective concentration range that can be applied at a varity of intervals. In some cases, it is preferred to apply equol in a concentration of up to 5% to treat some pathological conditions or diseases. There are also instances in which a concentration of up to 10% may be required, due to the severity of a condition or disease, or because an individual is a non-equol producer, thus requiring administration of a greater amount of exogenous equol.

A topical composition of the present invention can include other cosmetic and pharmaceutical actives and excipients. Such suitable cosmetic and pharmaceutical agents include, but are not limited to, antifungals, vitamins, anti-inflammatory agents, antimicrobials, analgesics, nitric oxide synthase inhibitors, insect repellents, self-tanning agents, surfactants, moisturizers, stabilizers, preservatives, antiseptics, thickeners, lubricants, humectants, chelating agents, skin penetration enhancers, emollients, fragrances and colorants.

In some individuals it is preferred to use a combination of systemic and topical administration. This can be due to the severity of the condition or disease, or because an individual is a non-equol producer, thus requiring administration of a greater amount of exogenous equol

An enantiomeric equol can also be an enantiomeric equol conjugate, conjugated at the C-4' or the C-7 position with a conjugate selected from the group consisting of glucuronide, sulfate, acetate, propionate, glucoside, acetyl-glucoside, malonylglucoside, and mixtures thereof.

A composition or preparation comprising enantiomeric or mixture of equol, for administering to subjects for the treatment and/or prevention of, or for reducing the predisposition to, androgen-related diseases and conditions related thereto, can also comprise one or more pharmaceutically acceptable adjuvants, carriers and/or excipients. Pharmaceutically acceptable adjuvants, carriers and/or excipients are well known in the art, for example as described in the Handbook of Pharmaceutical Excipients, second edition, American Pharmaceutical Association, 1994 (incorporated herein by reference). The composition can be administered in the form of tablets, capsules, powders for reconstitution, syrups, food (such as food bars, biscuits, snack foods and other standard food forms well known in the art), or in drink formulations. Drinks can contain flavoring, buffers and the like.

The composition can comprise a non-racemic mixture of S-equol and R-equol, having an enantiomeric excess (EE) for S-equol of more than 0% and less than 90%. A composition that has an EE of 0% is a 50:50 racemic mixture of the two enantiomers. The composition can be made directly from a racemic mixture, by an incomplete separation and removal of either the R-equol or S-equol enantiomer from the racemic mixture. The composition can also be made by combining a first equol component comprising a mixture (either a non-racemic or racemic mixture) of equol, with a second component comprising a composition consisting essentially of S-equol or R-equol. This produces a non-racemic composition that has an excess of S-equol or R-equol. Depending upon the specific benefit or indication for the R-equol component and the S-equol component in a composition, a composition can be prepared comprising S-equol and R-equol at a ratio of S-equol to R-equol from greater than about 50:50 to about 99.5:1, more typically about 51:49 to about 99:1,and from less than about 50:50 to about 1:99.5, more typically about 49:51 to about 1:99. The composition typically does not comprise a significant amount of any other androgen-receptor binding compound. Selected ratios of S-equol to R-equol includes from about 3:1 to about 19:1, about 3:1 to about 9:1, about 4:1 to about 19:1, and about 4:1 to about 9:1.

Compositions suitable for oral administration can be presented in discrete units, such as capsules, cachets, lozenges, or tablets, each containing a predetermined amount of the extract; as a powder or granules; as a solution or a suspension in an aqueous or non-aqueous liquid; or as an oil-in-water or water-in-oil emulsion.

A fuller understanding of the invention and its uses can be derived from the following experiments.

Experiment 1. Determination of receptor binding capacity of equol S- and R-enantiomers. *In vitro* binding studies were performed to examine the relative affinities of S- and R-enantiomeric equol with the estrogen receptors ERα and ERβ. Full length rat ERα expression vector (pcDNA-ERα; RH Price UCSF) and ERβ expression vector (pcDNA-ERβ; TA Brown, Pfizer, Groton, CT) were used to synthesize hormone receptors in vitro using the TnT-coupled reticulocyte lysate system (Promega, Madison, WI) with T7-RNA polymerase, during a 90 min reaction at 30° C. Translation reaction mixtures were stored at -80° C until further use. In_order to calculate and establish the binding affinity of the S-equol and R-equol enantiomers for ERα and ERβ, 100μL aliquots of reticulocyte lysate supernatant were incubated at optimal time and temperature; 90 min at room temperature (ERβ) or 18 hrs at 4°C (ERα), with increasing (0.01-100 nm) concentrations of [$^3$H] 17β-estradiol (E2). These times were determined empirically and represent optimal binding of receptor with estrogen. Nonspecific binding was assessed using a 300-fold excess of the ER agonist, diethylstilbestrol, in parallel tubes. Following incubation, bound and unbound [$^3$H]E2 were separated by passing the incubation reaction through a 1mL lipophilic Sephadex LH-20 (Sigma-Aldrich Co., Saint Louis, MO) column. Columns were constructed by packing a disposable pipette tip (1mL; Labcraft, Curtin Matheson Scientific, Inc, Houston, TX) with TEGMD (10mm Tris-Cl, 1.5 mm EDTA, 10% glycerol, 25mm molybdate, and 1mm dithiothreitol, pH 7.4)-saturated Sephadex according to previously published protocols (Handa et al., 1986; O'Keefe and Handa, 1990). For chromatography, the columns were re-equilibrated with TEGMD (100μL), and the incubation reactions were added individually to each column and allowed to incubate on the column for an additional 30 min. Following this incubation, 600μL of TEGMD were added to each column, flow-through was collected, 4 mL scintillation fluid was added, and samples were counted (5 min each) in an 2900 TR Packard scintillation counter (Packard Bioscience, Meriden, CT).

Competition binding studies were used to assess the estrogenic properties of equol's S-equol and R-equol enantiomers. Based on the ability of S and R to compete with [$^3$H]E2 for ER binding, the affinities for in vitro translated ER were shown to be very different for the two enantiomers. The S-equol enantiomer showed greatest affinity for ERβ [Kd (nM) = 0.73 ± 0.2], while its affinity for ERα was relatively low by comparison [Kd(nM) = 6.41 ± 1.0]. The R-equol enantiomer possessed a much lower affinity for both ERβ [Kd (nM) = 15.4 ± 1.3] and ERα [Kd (nM) = 27.38 ± 3.8]. For reference 17β-estradiol binds ERα with a Kd (nM) = 0.13 and ERβ with a Kd (nM) = 0.15 in this system.

The study showed that only the S-equol enantiomer binds ER with sufficient affinity to have potential relevance to circulating equol levels reported in humans. Compared with 17β-estradiol the relative binding affinities of the S-equol and R-equol enantiomers for ERα were 1/49 and 1/211that of 17β-estradiol, respectively. However, the S-equol enantiomer seems to be largely ERβ-selective with a relatively high affinity for ERβ, while the R-equol enantiomer binds with approximately 1/100 the affinity of S-equol. The separate and associated determination that exclusively S-equol is found in human plasma and urine is significant in view of the specificity in binding of the two enantiomers.

Experiment 2. Bioavailability of R-equol: 20 mg of pure R-equol was administered orally to a healthy adult after an overnight fast. Blood samples were collected at timed intervals over the next 24 hours and the plasma concentration of equol was determined by isotope dilution gas chromatography-mass spectrometry with selected ion monitoring. Rapid appearance of equol is observed in the plasma with peak concentrations observed after 8 hours. The terminal elimination half-life of R-equol was approximately 8 hours. Electrospray ionization mass spectrometry confirmed that the equol present in plasma was the R-equol enantiomer (PCT Patent Publication W004-009035), thereby establishing that it is stable and does not undergo any racemization or further biotransformation in the intestine. FIGURE 2 shows a plasma appearance/disappearance plot of R-equol. These results establish that R-equol, if administered as a pharmacologic or nutraceutical preparation, is bioavailable. Similar results have been obtained where S-equol was administered orally to a healthy adult.

EXAMPLES

The following examples demonstrate the use of the invention, and the benefits that are derived from it. In order to demonstrate the efficacy of the invention, the following protocols are used, and will be referred to in the examples that follow:

Protocols Used in Examples of the Invention

1. Preparation of Test Material Stock Solutions

Approximately 20 to 40 mg of the test material was weighed into pre-tared sterile glass vials and the precise weight was recorded. Vehicle volume is then calculated to give a 50% w/v solution, and the vehicle is added. Two different vehicles were used, as indicated in Examples. DMSO vehicle is prepared from 100% DMSO (EMD Biosciences Cat. #MX1458-6, Lot #42364321). Transcutol vehicle, prepared from 100% transcutol (Gattefosse a.s.a., Cedex, France). Samples are then vortexed vigorously until the dry powder is visually brought into solution. In some cases, samples need to be briefly warmed to 37° C. Stock solution aliquots can be frozen in small aliquots and maintained at approximately -20° C, or used immediately after preparation. Racemic mixtures of equol are prepared for testing at concentrations of 0 (control), 0.3, 1.0, and 5.0%. A positive control is prepared using ascorbic acid at a concentration of 50 μg/ml in DMEM/F-12. After stock solutions are diluted for use they are then discarded.

2. MTT Assay

MTT Assay is performed to determine toxicity of equol in tissue culture. Measurement of cell viability and proliferation forms the basis for numerous in vitro assays of a cell population's response to external factors. The reduction of tetrazolium salts is now widely accepted as a reliable way to examine cell proliferation. The MTT assay is a colorimetric analysis of the metabolic activity of the cell (ATCC; Gaithersberg MD; Catalogue #30-1010K). The yellow tetrazolium MTT (3-(4, 5-dimethylthiazolyl-2)-2, 5-diphenyltetrazolium bromide) is reduced by metabolically active cells to generate reducing equivalents such as NADH and NADPH. The resulting intracellular purple formazan can be solubilized and quantified by spectrophotometric means. The MTT Cell Proliferation Assay measures the cell proliferation rate and conversely, when metabolic events lead to apoptosis or necrosis, the reduction in cell viability. A linear relationship between cell number and signal produced is established, thus allowing an accurate quantification of changes in the rate of cell proliferation. In some cases or experiments, the results of the MTT Assay will be used to normalize the results of the Procollagen Type I C Peptide Assay.

A compatibility test is carried out with the test material(s) used in the test system. An aliquot of each test material is mixed with an equal volume of 2 mg/ml MTT solution, in a glass test tube. Tubes are capped and incubated in the dark at room temperature for approximately 2 hours unless conversion occurs sooner. Evidence of a color change to purple indicates that the test material may spontaneously reduce MTT, resulting in a false reaction. If a color change is noticed, "blank tissue meshes" are run. These blanks are dosed with the same amount of test material as in the assay and run for the longest time point only. Background readings are subtracted from the respective test materials if any are observed.

3. Use of TestSkin in Tissue Culture

TestSkin II tissue is used to assess the ability of the test materials to either promote or inhibit collagen synthesis. This test is also used to assess the viability of the tissues after exposure to the test materials. TestSkin II consists of a mechanically stable and physiologically functional skin construct that closely simulates the actual structure and biological response mechanism of living human skin. The tissue has both an epidermis and a dermis. The upper epidermal layer consists of living human keratinocytes and has a well differentiated stratum corneum. The epidermis grows on a supporting dermal layer that consists of a bovine collagen lattice interspersed with living human dermal fibroblasts that align the collagen into a dense matrix. TestSkin is used according to the following Tissue Culture Protocol

TestSkin Tissue Culture Protocol, Day One:

Enough six-well plates are taken to complete the assay and placed under the hood. The package containing each plate is opened, the plate cover removed and turned upside down on the working surface. One Millicell per well is placed into each well of a six-well plate, along with 1 ml of DMEM/F-12. The plate cover is replaced and the six-well plate set aside until the TestSkin II unit is sectioned and ready for storage. To section the TestSkin II unit, the outer surface of the sealed pouch is wiped with 70% ethanol. The pouch is opened using sterile scissors and the tray containing the TestSkin II unit removed. The cover from the TestSkin II tray is removed and placed upside down on the working surface, so that the inside of the cover remains sterile. The TestSkin II transwell is removed by using sterile forceps and placed on the inside of the cover. To section the TestSkin II unit, a sterile biopsy punch (8mm) is used. The punch is placed on the surface of the TcstSkin II unit and slowly pressed down and twisted simultaneously. The punch is rotated back and forth approximately ¼ turn while sufficient pressure is applied to cut through both the skin and the polycarbonate membrane. The punch is not twisted completely around, as this tends to rip the skin. When the individual section is separated from the rest of the skin, it is removed with sterile needle nose forceps; only the edge of the section is grasped, and care is taken not to pinch or fold the section. The section is carefully placed dermis side down onto the Millicell, ensuring that it lies flat with no air bubbles between the bottom of the Millicell and the skin. It is helpful for one edge to be placed down first and the remaining portion slowly lowered so that no air bubbles form. The epidermal surface remains facing up and the polycarbonate membrane with the dermal surface lies against the Millicell. This procedure is repeated until the correct number of sections have been separated and placed within the Millicells of the six-well plate. Once the sectioning is complete, the cover is placed back onto the plate and the plate is placed into an incubator at approximately 37±2°C and 5±1% $CO_2$ for overnight (16-24 hours). TestSkin Tissue Culture Protocol, Day Two:

The media in each well is aspirated and replaced with 1 ml fresh DMEM/F-12 media. For each treatment group (negative control, positive control, and test materials), 10 μl of material is placed onto tissues and covered with a dosing pad. The tissues are placed back into the incubator at approximately 37±2°C and 5±1% $CO_2$ for approximately 48 hours.

TestSkin Tissue Culture Protocol, Day Three:

All test samples are dosed topically again with 10 μl of each treatment per tissue. The tissues are placed back into the incubator at approximately 37 ± 2°C and 5 ± 1% $CO_2$ for approximately 24 hours.

TestSkin Tissue Culture Protocol, Day Four:

The media from each well is removed and frozen for subsequent procollagen assays. The MTT assay is performed immediately on three of the four tissue samples in each treatment set. The fourth tissue from each treatment group is fixed in formalin, paraffin-embedded, sectioned, and subjected to histological staining. The histology slides are examined by microscope for elastin and collagen analysis.

A 2 mg/ml MTT solution (enough for 2 mg/tissue) is made with DMEM/F-12 (pre-warmed to 37 ± 2°C). The MTT solution is mixed for 10-15 minutes at room temperature on a stir plate. The solution is then centrifuged for 5 minutes at 4000 rpm. The pellet is discarded and only the supernatant used. The MTT solution is added to the wells of a six-well plate (2 ml/well). The tissues are removed from the Millicell inserts and rinsed with at least 5 ml of PBS from a wash bottle over a beaker. The tissues are rinsed until all test material is removed. The tissues are then placed into the corresponding well of the six-well plate. The plates are incubated for approximately 2 hours at approximately 37±2°C and 5±1% $CO_2$ on a shaker plate at 125 rpm. At the end of the 2 hr incubation, the MTT solution is removed and discarded. One ml of PBS is added to each well for 2 minutes twice. Each PBS wash is removed by aspiration.

After the MTT exposure, the tissues are incubated in a 600 μg/m1 Thermolysin solution (DMEM/F-12 for a 2 ml total volume) for 30 minutes at 37±2°C. This incubation time should be sufficient to allow for the separation of the dermal and epidermal layers of the tissue. The epidermis should be floating on top of the dermis. The dermal portions of the tissue are placed into a separate set of 6-well plates. The MTT is extracted from both sets of plates with 1 ml per well of isopropyl alcohol. The plates are placed on a shaker plate for 1 hour. After isopropanol extraction, 200 μl of extract is transferred to the corresponding wells of a 96-well plate. The plate is read at 540 nm.

4. Human Dermal Fibroblast Tissue Culture Protocol

Primary human dermal fibroblasts from neonatal foreskin at passage 10-11 were seeded into 48 well plates at 2.5e4 cells/well/0.5 mls medium which consisted of DMEM (MediaTech, Cat. #10-017-CV, Lot #10017103) with 1x non-essential amino acids (HyClone Cat. #SH30238.01, Lot #AMC15759), 1x antibiotic/antimycotic (Sigma Cat. # A5955, Lot#13K2363) and 2% bovine calf serum (HyClone Cat. #SH30072.03, Lot #ANF-18955). Samples from a previous lot of calf serum (Lot # AMM17780), were also used. Cells were cultured for approximately 16-24 hours in a 37° C, humidified incubator with 5% $CO_2$, then the medium was changed and equol, ascorbic acid (Sigma, Cat. #A4544, Lot#073K0139) or vehicle was added to media in the wells. Cells were cultured for approximately 48 hours in the presence of test or control materials.

5. Organotypic, Three-dimensional Dermal Cultures

For the production of organotypic, three-dimensional (3D) cultures, dermal fibroblasts were seeded to nylon mesh and allowed to grow for approximately 8 weeks essentially as described (see Fleishmajer, J Invest Dermatol, 97: 638-643, 1991; Contard, Cell Tissue Res, 273: 571-575, 1993 and Pinney, Liu, Sheeman and Mansbridge, J Cell Physiology, 183: 74-82, 2000). This *in vitro* model closely mimics the development of the dermis, offering a system for study with organotypic properties, such as the ability to support epidermal differentiation (see Slivka, J Invest Dermatol, 100: 40-46, 1993) and collagen fibrillogenesis (Contard, Cell Tissue Res, 273: 571-575, 1993). After 2 weeks all 3D cultures were supplemented with 20 ug/ml ascorbate while monolayer cultures did not. Otherwise, all materials and procedures were essentially equivalent between monolayer and 3D experiments. To examine the effects of estrogenic test materials in an environment with undetectable levels of estrogenic activity, the 3D experiments were grown in phenol red-free medium for 3 weeks prior to addition of test materials, and were therefore never exposed to the phenol red dye.

6. Human Prostate Cancer Cell Cultures

Human prostate cancer cell line was obtained from ATCC (ATCC # CRL-1740, LNCAP-FGC) and was cultured in a 37° C, humidified incubator with 5% $CO_2$, in RPMI Medium (Sigma Cat. # R-8758) with 5% fetal bovine serum (Hyclone Cat. # SH30088.03, Lot number APC20780) and 5mM Hepes (Sigma Cat. H-0887), 1x antibiotic/antimycotic (Sigma Cat. # A5955). Cells were expanded in T-150 flasks for three passages until cryopreservation and storage in liquid nitrogen in RPMI medium with 10% FBS and 10% DMSO as cryopreservative. A cryovial was then thawed in a 37° C water bath, expanded again one or two passages, and then plated at 10,000 cells per 96 well in 0.2 mls medium in 96 well plates in RPMI 5% FBS medium. After approximately 48 hours, the medium was changed to phenol red-free DMEM/F12 (Gibco Cat. #21041-025) with 2% FBS and 1x antibiotic/antimycotic. and test materials and DMSO/vehicle controls were added to the appropriate concentration from 10x stocks. Cells were cultured for approximately 48 hours in the presence of test materials and controls prior to removal of medium supernatants for prostate specific antigen (PSA) ELISA.

7. Collagen Type I C-Propeptide ELISA

Collagens (types I, II, III, IV, and V) are synthesized as precursor molecules called procollagens. These contain additional peptide sequences called propeptides that facilitate the winding of procollagen molecules into a triple-helical conformation within the endoplasmic reticulum. The propeptides are cleaved off from the collagen triple helix molecule during its secretion, after which, the triple helix collagens polymerize into extracellular collagen fibrils. Thus, the amount of the free propeptides reflects stoichiometrically the amount of collagen molecules synthesized (Takara Biomedicals, Collagen Type I C-Propeptide Kit).

Dermal fibroblasts synthesize primarily type I collagen, and the cleavage of the C-terminal propeptide is required for deposition into fibrils within the extracellular matrix. This propeptide can be measured using antisera which do not recognize the unprocessed form in cell culture supernatants, and is also used clinically as a measure of fibrosis in patient sera. The amount of cleaved propeptide is directly proportional to the amount of type I collagen, deposited, and can be precisely quantified using purified standards and a commercial ELISA kit (Takara Mirus, Inc., Cat. #TAK-MK-101). After 48 hours in the presence of test materials or controls, culture medium supernatants were removed and immediately analyzed using the ELISA kit according to manufacturer's instructions using a Molecular Devices Vmax plate 96 well plate reader and SoftMax software. Since ascorbic acid (ascorbate, Sigma Cat. #A4544, Lot #073K0139) is known to stimulate collagen deposition, it was used as a positive control, and added to media in a final concentration of 20 ug,/ml. Vehicle-treated media or media alone was used as a negative or blank control.

Supernatants or media from tissue culture is collected from culture plates or wells. Sample supernatants are spun in a centrifuge at 2000-3000 rpm for 5-10 minutes and collagen Type I C-propeptide is quantitated. The pellet is not used in the study. The preparation of the assay solutions is as follows:

Standard Solution (640 no PIP/mi):

The standard is rehydrated with 1 ml of distilled water and mixed slowly by rolling on the countertop intermittently for approximately 10 minutes. The standards are made as shown in TABLE 1. The standard solution is stable for 1 week at 4°C. The standards are tested in duplicate.

TABLE 1. Standard Solutions for Procollagen Type I C-Peptide Assay.

| | Standard Assy Solutions | | | | | | | |
|---|---|---|---|---|---|---|---|---|
| Final Cone. (ng/ml) | 0 | 10 | 20 | 40 | 80 | 160 | 320 | 640 |
| Maintenance Media (μl) | 400 | 394 | 388 | 375 | 350 | 300 | 200 | 0 |
| Standard Solution (μl) | 0 | 6 | 12 | 25 | 50 | 100 | 200 | 400 |

Stop Solution (1N $H_2SO_4$):

5.8 ml of concentrated $H_2SO_4$ is carefully added to 180 ml of distilled water. Distilled water is added to give a final volume of 200 ml. The solution is mixed well. This solution can be stored at 2-26°C for up to 6 months.

Antibody-POD Conjugate Solution:

The contents of Vial 1 are dissolved in 11 ml of distilled water and mixed gently by rolling on the countertop. The vial must be wrapped in foil as it is light sensitive. The solution is mixed slowly for approximately 10 minutes and foam formation is avoided. This solution is prepared directly before use and transferred immediately to the microtiter plate. Aliquots of 100 μl of antibody-POD Conjugate Solution are pipetted into the standard wells and the sample wells. The standard and the samples (20 μl of each) are pipetted into their corresponding wells. The microtiter plate is mixed by gently tapping the side for 15 seconds, then is sealed with foil and incubated at 37°C for approximately 3 hours. At the end of the incubation, the solutions are removed from each well by inverting the plate, and washing each well 4 times with approximately 400 μl of PBS. Between each wash, the microtiter plate is emptied by inverting it and tapping upside down on a paper towel to remove as much of the PBS as possible. Substrate solution (100 μl) is pipetted into each well. The plate is tapped gently for 15 seconds to mix, and incubated at room temperature (20-30°C) for 15 minutes. Stop solution (100 μl) is pipetted into each well and the plate tapped gently to mix for 15 seconds. The absorbance of the samples is measured at 450 nm on the ELISA microplate reader.

Calculations and Data Analysis:

For the Collagen Type I C-Propeptide assay, if more than one reading per sample is taken, the readings for each sample are averaged. To derive the standard curve, plot the absorbance versus the PIP concentration in ng/ml for the standards using log-log scale. For the samples, locate the average absorbance value on the vertical axis and follow a horizontal line intersecting the standard curve. At the point of intersection, read the PIP concentration (ng/ml) from the horizontal axis. The procollagen values (ng) are normalized for variations in tissue size or makeup by dividing by the dermis MTT value, since the dermal layer of the tissue is responsible for collagen synthesis.

The mean OD value and standard deviation for all MTT (dermis + epidermis) replicate samples is calculated. The percent of viability is calculated by using the following equation:

$$\text{Viability (\%)} = (\text{Mean OD of Test Material / Mean OD of Negative Control}) \times 100.$$

8. PSA ELISA

Tissue culture supernatants were diluted 10-fold in PBS and stored at -20°C, then thawed at room temperature prior to assaying. A commercial ELISA kit for free PSA (Bio-Quant, Cat. # BQ 067T) was utilized according to the manufacturer's instructions, and data was determined using a Molecular Devices Vmax 96 well plate reader and SoftMax software.

9. Intracellular FACS Analysis and Cell Cycle Determinations

Single-cell suspensions were produced by gently trypsinization of monolayers, or extensive digestion in 1 mg/ml collagenase from 3D cultures. A commercial kit was utilized for the preparation of cells for intracellular detection by flow cytometry (IntraCyte, Orion BioSolutions, Inc., Cat. # 01017) according to the manufacturer's instructions. In brief, cells were fixed with formaldehyde, permeabilized with non-ionic detergents, and non-specific protein binding was blocked. The following primary antibodies were used at 1-2 ug/ml: affinity-purified, anti-hunan collagen type I (Chemicon Inc., Cat. #AB758), affinity-purified anti-human type III collagen (Sothern Biotechnology Associates, Inc. Cat. # 1330-01), monoclonal anti-human elastin (Sigma, Inc., Cat. # E4013), polyclonal anti-human elastase (The Binding Site Inc., Cat. #PC052), and monoclonal anti-human MMP-3/stromelysin-l (Calbiochem Inc., Cat. #IM362). Negative controls included irrelevant immunoglobulins from the same species as each primary antibody and at the same concentration, as well as unstained cell, and cells without primary antibody. Primary antibody binding was detected using affinity-purified, species-specific, fluorochrome-conjugated secondary antibodies. For FACS analysis, a Coulter EPICS Elite cytometer equipped with 488 nm argon laser was used and approximately 20,000 cells per file were analyzed using Coulter ELITE software.

10. Ocular and Dermal Irritection Assay

The Ocular and Dermal Irritection assays are quantitative *in vitro* test methods that mimic acute ocular and dermal irritation tests. To perform the Ocular Irritection standardized assay, the test sample is applied to a synthetic biobarrier composed of a semi-permeable membrane. To perform the Dermal Irritection standardized assay, the test sample is applied to a similar synthetic biobarrier that is coated with a dye-containing keratin-collagen matrix. Following application, the sample is absorbed by and permeates through this synthetic biobarrier to gradually come into contact with a proprietary solution containing highly ordered globulins and glycoproteins. Reaction of the test sample with these proteins and macromolecular complexes promotes conformational changes that may be readily detected as an increase in the turbidity of the protein solution. With the Ocular Irritection test, turbidity may be detected spectrophotometrically at a wavelength of 405 mn. With the Dermal Irritection test, dye that has been dissociated from the biobarrier during transit of the applied sample may be detected spectrophotometrically at a wavelength of 450 450 mn.

The ocular irritancy potential of a test sample is expressed as an Irritection Draize Equivalent (IDE), whereas the dermal irritancy potential of a test sample is expressed as a Human Irritancy Equivalent (HIE) score. These scores are defined by comparing the increase in optical density ($OD_{405/450}$) produced by the test material to a standard curve that is constructed by measuring the increase in OD produced by a set of Calibration substances. These Calibrators have been selected for use in these tests because their irritancy potential has been previously documented in a series of *in vivo* investigations. The predicted *in vivo* classification, based on these scoring systems, is shown in TABLES 2 and 3. In general, the program has been designed to accept sample data as qualified if the following criteria are met: the OD values of Calibrators and internal Quality Control samples fall within previously specified ranges; sample blanks are less than 500 OD units; the net sample OD is greater than -15; and an Inhibition Check is negative.

TABLE 2. Relationship of Irritection Draize Equivalent (IDE) Score to Irritancy Classification for the Ocular Irritection Test Method.

| Irritection Draize Equivalent (IDE) Score | Predicted Ocular Irritancy Classification |
|---|---|
| 0.0-12.5 | Minimal Irritant |
| 12.5 - 30.0 | Mild Irritant |
| 30.0-51.0 | Moderate Irritant |
| 51.0-80.0 | Severe Irritant |

TABLE 3. Relationship of Human Irritancy Equivalent (HIE) Score to Irritancy Classification for the Dermal Irritection Test Method.

| Human Irritancy Equivalent (HIE) | Predicted Dermal Irritancy Classification |
|---|---|
| 0.00-0.90 | Non-Irritant |
| 0.90-1.20 | Non-Irritant/Irritant |
| 1.20-5.00 | Irritant |

11. Statistical Analyses

Where appropriate, data were analyzed by analysis of variance statistics (ANOVA) followed by Newman-Keuls post hoc tests. Significance was $p < 0.05$. Curve fitting, scientific graphing, and analysis were completed using GraphPad Software(GraphPad Prism 3.0, San Diego, CA).

Example 1:

This example demonstrates equol selectively binding *in vitro* to 5α-DHT. In initial binding competition studies conducted to determine and establish equol's binding affinity for AR, binding of [$^3$H] 5α-DHT was greater in the presence of equol than in its absence. Slight modifications in the protocol where AR was removed from the incubation tube (leaving only [$^3$H] 5α-DHT and equol) resulted in the elution of [$^3$H] 5α-DHT into the eluate containing [$^3$H) 5α-DHT reaction complex. Sephadex LH-20 columns of 30 cm are used in order to identify elution peaks establishing the binding of [$^3$H] 5α-DHT to equol. As shown in FIGURE 3, a peak of [$^3$H] 5α-DHT is apparent in the elution fractions between 5 and 9mL when the [$^3$H] 5α-DHT+equol column incubate is applied. This peak is not present when [$^3$H] 5α-DHT alone is applied to the column. Furthermore, when 5α-DHT or 5α-DHT+equol are incubated with prostate supernatant and then passed through the 30cm column (FIGURE 4A) two distinct binding peaks are identifiable. The first peak of [$^3$H] 5α-DHT represents that bound to the AR in prostate. This is found in the elution fractions between 4 and 5 ml. In addition there is a later peak (between 5 and 9 ml), consistent with the binding of [$^3$H] 5α-DHT to equol. However, when [$^3$H] 5α-DHT is allowed to incubate with the prostate supernatant for 36 hours (until equilibrium) prior to the introduction of equol there is no apparent binding of [$^3$H] 5α-DHT (FIGURE 4B). Both [$^3$H] 5α-DHT and [$^3$H] 5α-DHT + equol (equol added 36 hours later) show a single peak in the elution between 4 and 5ml, suggesting that equol does not compete with 5α-DHT for the AR nor does it bind [$^3$H] 5α-DHT that is already bound to the receptor. Furthermore, it should be noted that the binding of equol to 5α-DHT appears to be specific, since similar competition and binding studies have been conducted using other steroids such as [$^3$H]E2, [$^3$H)T, [$^3$H]DHEA, [$^3$H]CORT and [$^3$H]progesterone without any occurrences of significant binding to equol (data not shown). Saturation analysis of equol binding to [$^3$H] 5α-DHT shows an apparent Kd calculated at 1.32 ± 0.4 nM (FIGURE 5).

TABLE 4 shows 33 different steroid compounds that have been tested in binding assays to determine equol's affinity for binding to each. While equol has modest affinity for 5α-reduced steroids, equol displayed the highest affinity for 5α-dihydrotestosterone (5α-DHT) and had no affinity for 5β-dihydrotestosterone (5β-DHT) or some of the most common natural sex steroids, such as: estradiol, estrone, estriol, progesterone or testosterone.

TABLE 4 Steroid Compounds used in Equol Binding Assays

| Chemical Name | Trivial Name |
|---|---|
| 4-ANDROSTEN-17β-OL-3-ONE | TESTOSTERONE |
| 5-ANDROSTEN-3β-OL-17-ONE | DHEA |
| 5α-ANDROSTAN-3α,17β-DIOL | 17β-DIHYDROANDROSTERONE |
| 5α-ANDROSTAN-3β-17α-DIOL | NA |
| 5α-ANDROSTAN-3β,17β-DIOL | NA |
| 5α-ANDROSTAN-3,17-DIONE | ANDROSTANEDIONE |
| 5α-ANDROSTAN-17β-OL-3-ONE | 5α-DHT |
| 5β-ANDROSTAN-17β-OL | NA |
| 5β-ANDROSTAN-17β-OL-3-ONE | 5β-DHT |
| 4-ANDROSTEN-3,7-DIONE | ANDROSTENEDIONE |
| 1,3,5(10)-ESTRATRIEN-3,17α∀-DIOL | EPIESTRADIOL |
| 1,3,5(10)-ESTRATRIEN-3,17β-DIOL | ESTRADIOL (E2) |
| 1,3,5(10)-ESTRATRIEN-3,16α,17β-TRIOL | ESTRIOL (E3) |
| 1,3,5(10)-ESTRATRIEN-3-OL-17-ONE | ESTRONE (E1) |
| 5α-ESTRAN-3,17-DIONE | 5α-DIHYDROANDROSTENDIONE |
| 5-PREGNEN-3β-OL-20-ONE | PREGNENOLONE |

(continued)

| Chemical Name | Trivial Name |
|---|---|
| 4-PREGENEN-3,20-DIONE | PROGESTERONE (P4) |
| 5α-PREGNAN-3α-OL-20-ONE | ALLOPREGNANOLONE |
| 5α-PREGNAN-11β,21-DIOL-3,20-DIONE | ALLODIHYDROCOSTERONE |
| 5α-PREGNAN-3α,11β,21-TRIOL-20-ONE | ALLOTETRAHYDROCORTICOSTEROME |
| 5α-PREGNAN-3β, 21β, 21-TRIOL-20-ONE | EPIALLOTETRAHYDROCORTICOSTERONE |
| 5α-PREGNAN-3β,11β,17,21-TETROL-20-ONE | 3β,5α-TETRAHYDROCORTISOL |
| 5α-PREGNAN-11β,17,21-TRIOL-3,20-DIONE | ALLODIHYDROCORTISOL |
| 5α-PREGNAN-17,21-DIOL-3,11,20-TRIONE | ALLODIHYDROCORTISONE, |
| 5α-PREGNAN-3β-OL-20-ONE | 5α-DIHYDROPREGNANOLONE |
| 5α-PREGNAN-3,20-DIONE | 5α-DIHYDROPROGESTERONE (5α-DHP) |
| 5β-PREGNAN-3α-OL-20-ONE | 3α-HYDROXY-5βTETRAHYDROPROGESTONE |
| 5β-PREGNAN-11β,21-DIOL-3,20-DIONE | 5β -DIHYDROCORTICOSTERONE |
| 5β-PREGNAN-3α,11β,21-TRIOL-20-ONE | TETRAHYDROCORTICOSTERONE |
| 5β-PREGNAN-3α,11β,17,21-TETROL-20-ONE | TETRAHYDROHYDROCORTISOL |
| 5β-PREGNAN-11β,17,21-TRIOL-3,20-DIONE | 5β-DIHYDROCORTISOL |
| 5α-PREGNAN-17,21-DIOL-3,11,20-TRIONE | 5β -DIHYDROCORTISONE |
| 5β-PREGNAN-3,20-DIONE | 5β-DIHYDROPROGESTERONE (5β-DHP) |

Example 2:

Long-Evans male rats are raised on either a phytoestrogen-rich diet containing 600 micrograms of isoflavones per gram of diet or 600 ppm of isoflavones (referred to hereafter as the "Phyto-600" diet) or a diet containing very low levels of isoflavones (referred to hereafter as the 'Phyto-Free' diet; containing approximately 10 ppm of isoflavones). To demonstrate that circulating isoflavone levels are different in Phyto-600- vs. Phyto-Free-fed male and female (75 day-old) rats, serum isoflavone levels were determined by GC/MS as previously performed by our laboratories (see methods in K.D.R. Setchell, Am J Clin Nutr 129:1333S-1346S, 1998; and K.D.R. Setchell et al, J Nutr 132:3577-3584, 2002.). In each case for the different classifications of isoflavones Phyto-600-fed males display significantly higher isoflavones levels compared to Phyto-Free-fed values, shown in TABLE 5 as isoflavone concentrations in adult male and female rats. More importantly, equol levels in the Phyto-600-fed rats account for approximately 78% of the total phytoestrogen levels.

TABLE 5. Isoflavone levels in male rats (ng/ml serum)

| | Genistein | Daidzein | Equol | Total |
|---|---|---|---|---|
| - Phyto-Free Diet | 9.6 ±0.3 | 10.8 ±0.6 | 23.2±0.4. | 43.5±1.0 |
| - Phyto-600 Diet | 413 ±67 | 394 ±58 | 1,161 ± 325 | 1,967 ±45 |

To determine if other metabolic hormones were altered by the diet treatments or by age, serum glucose and thyroid (T3) levels are assayed. Glucose levels are slightly but not significantly higher in the Phyto-600-fed males compared to Phyto-Free-fed values, independent of age or source of the blood samples [either arterial (ART) or venous (TRUNK)], shown in FIGURE 6. However, when T3 levels are quantified, there is a significant increase in T3 serum levels in 80 or 110 day-old male Long-Evans rats fed the Phyto-600 diet compared to Phyto-Free-fed animals, shown in FIGURE 7. This demonstrates that thyroid levels are enhanced with soy consumption, consistent with anecdotal evidence of individuals that decreased their thyroid medication or went off of thyroid treatment completely with the consumption of soy based foods in their diets. This is also consistent with reports of a similar increase in T3 levels in humans following consumption of soy foods (Watanabe, S. et al, Biofactors 2000: 12:233-41 and Lephart, E.D. et al, Nutrition Metab (London) 2004:1:16).

Example 3:

Prior to initiation of a Phyto-Free diet period, Male Long-Evans rats are fed a Phyto-200 diet, as described in previous examples. The rats are placed on a diet containing the Phyto-Free diet at approximately 52 days of age and randomly assigned to three groups. Beginning at 73 days of age, rats receive daily subcutaneous 0.1cc injections of vehicle (peanut oil), 1 milligram of a racemic mixture of equol in vehicle (0.83 mg/kg body weight/day), or 5 milligrams of a racemic mixture of equol in vehicle (4.2 mg/kg body weight/day) once every three days. To determine whether equol injections have an adverse effect on male reproductive organs, testis weights are quantified in these animals. There are no significant alterations in testes weight with the equol injections, with testicular weight essentially the same among the injection treatment groups, shown in FIGURE 8.

FIGURE 9 shows the distribution in human skin of estrogen receptor beta (ER-β), the 5α-reductase enzyme (5α,R) and androgen receptors (AR) in balding skin (FIGURE 9A), hair follicle bulb (FIGURE 9B) and sebaceious gland (FIGURE 9C). Familiarity with the locations of these enzymes and receptors is important for discussion of the following examples.

Example 4:

The effect of equol on collagen synthesis in the TestSkin model was assessed. In examining the epidermal region only, vehicle, ascorbic acid, or 0.3% racemic equol have similar amounts of procollagen as measured by the Procollagen Type I C-PeptideAssay, above, shown in FIGURE 10. The negative control substance synthesized less procollagen compared to the control vehicle. However, a 1.0% concentration of racemic equol induced approximately 4 times as much procollagen synthesis, and 5.0% racemic equol induced an 18-fold increase versus vehicle control levels.

The normalized average of epidermal and dermal regions treated with vehicle, ascorbic acid, or 0.3% racemic equol also demonstrates similar amounts of procollagen, shown in FIGURE 11, and samples incubated with the negative control substance synthesized less procollagen. Racemic equol at 1% concentration induced approximately 4 times as much procollagen synthesis, and a 5% concentration resulted in an approximate 6-fold increase. In examining the dermal region only vehicle, ascorbic acid, or 0.3% racemic equol have similar amounts of procollagen, shown in FIGURE 12, and the negative control substance synthesized slightly less procollagen. Racemic equol at 1% and 5% concentrations induced approximately 4 times as much procollagen synthesized compared to vehicle control levels. Thus, using this artificial *(in vitro)* skin model, a threshold of 1 % equol appears to be sufficient to provide maximal stimulation of procollagen in the dermal region.

Example 5:

This study evaluated the effects of racemic equol and 17β-Estradiol at 3 different concentrations (0.01 %, 0.001 % and 0.0001 %) on primary human dermal fibroblast viability by MTT Assay, and collagen deposition by Collagen Type I C-propeptide ELISA.

Equol was tested against the natural steroid hormone, 17-β estradiol to determine cytotoxicity to human dermal monolayer fibroblasts by quantifying reduced MTT as described in the MTT Assay Protocol above (FIGURE 13). The test materials, equol and 17-β estradiol were dissolved in dimethysulfoxide (DMSO), a common cell culture vehicle. The test materials were assayed at 0.01, 0.001 and 0.0001% equol in 0.2, 0.02 and 0.002% of DMSO as the vehicle. The range for untreated controls varied from 0.77 to 0.93 OD units. Over the range of concentrations tested equol was no more toxic to human dermal monolayer fibroblasts than 17-β estradiol. Additionally, at concentrations of 0.001% and 0.0001% of the test materials the reduced MTT values were within the range of the untreated controls, indicating that the cytotoxicity levels were equivalent to untreated control values. At the highest concentration of 0.01%, the cytotoxicity levels of the test materials were approximately 0.52 and 0.48 for equol and 17-β estradiol, respectively, which are acceptable results for *in vitro* assay conditions. Thus, at the concentrations tested, equol is not more toxic to human dermal monolayer fibroblasts compared to the natural steroid hormone, 17-β estradiol.

Collagen deposition was quantified in human dermal monolayer fibroblast by ELISA (FIGURE 14). The vehicle DMSO served as the negative control, while ascorbate without DMSO served as the positive control. When groups treated with 100 and 10 μg/ml equol were compared to the same concentrations of 17-β estradiol, significant 2.1-fold and 1.55-fold increases, respectively, in collagen deposition were observed. This example demonstrates that equol has significantly greater collagen stimulating properties compared to the natural steroid hormone, 17-β estradiol. The significant increase in collagen production in this human dermal monolayer fibroblast assay demonstrates that the stimulatory effect of equol on collagen provides an effective way to treat skin parameters such as mechanical, physical and photo-aging damage and the influence of biological aging on wrinkle formation in skin.

Example 6:

The effects of equol on skin, as assessed by collagen deposition are assessed by Procollagen Type I C-Peptide ELISA using human dermal monolayer fibroblasts in *vitro.* This study evaluated the effects of a commercially used skin penetrating agent, transcutol in delivering the test material equol in determining human dermal fibroblast viability by MTT Assay, and collagen deposition by Collagen Type I C-temninal Propeptide ELISA. Transcutol, or dipropylene glycol, has been proven safe and effective in the delivery of active ingredients of cosmetic applications for human skin health (Final report on the safety, assessment of butylenes glycol, hexylene glycol, ethoxydiglycol, and dipropylene glycol, Journal of the American College of Toxicology, Volume 4, Number 5, 1985, Mary Ann Liebert, Inc. Publishers). When human dermal monolayer fibrobast cytotoxicity was examined, the equol treatment at 0.0001 % (w/v of transcutol) was not significantly different compared to the 0.0002% transcutol vehicle or untreated controls (FIGURE 15).

Next, equol was tested in the human dermal monolayer fibroblast assay to determine whether it could stimulate collagen deposition. As shown in FIGURE 16, untreated control values were at 330 $\pm$ 30 ng/ml (horizontal line) and this level was similar to that of 0.002% transcutol vehicle. However, 0.0001% equol significantly stimulated collagen deposition 1.8-fold above untreated control levels and 1.6-fold above the transcutol vehicle levels, demonstrating that transcutol is an effective method for the delivery of equol to human dermal fibroblasts. Furthermore, treatment with equol did not differ from the positive control treatment with ascorbate, suggesting that 0.000 1% equol maximally stimulated collagen deposition in this *in vitro* human dermal monolayer fibroblast assay system. The significant stimulatory influence of equol on human dermal fibroblasts to significantly increase the deposition of collagen can address several important issues of human skin health such as applications of mechanical, physical and photo-aging damage, and the natural biological and chronological process of skin aging.

Example 7:

This is an example of the ability of equol to bind and block 5α-DHT *in vitro.* Cytotoxicity of equol on human dermal fibroblasts cells was determined by MTT Assay (FIGURE 17). The negative control consisted of tissue medium (DMEM/HAMS F-12) alone with no DMSO, which yielded cell viability levels of approximately 0.83. This negative control was similar to the positive control, ascorbate which contained no DMSO, at approximately 0.85. Equol at 0.001 % (or 10 micrograms/ml in 0.002 % DMSO as the vehicle) displayed viability levels in the human dermal monolayer fibroblasts similar to that of the negative (no DMSO) and positive (ascorbate) controls. However, the potent natural androgen steroid hormone, 5α-DHT displayed the greatest cytotoxicity compared to all other treatment groups. When equol was added to the 5α-DHT samples *in vitro,* equol completely reversed the cytotoxic effects of 5α-DHT. This provides direct evidence that equol binds 5α-DHT in this *in vitro* human dermal monolayer fibroblast assay system and validates the *in vivo* evidence of equol biologically inactivating 5α-DHT's effects. Finally, by blocking the harmful effects of 5α-DHT in human dermal fibroblasts (cytotoxicity) and at the same time stimulating collagen deposition, this example demonstrates that equol has powerful effects on enhancing human skin health.

Example 8:

This is an example of the effects of equol preventing the stimulatory effects of 5α-DHT in LNCAP prostate cancer cells *in vitro* from secreting prostate-specific antigen (PSA), a molecule known to be regulated by 5α-DHT, as measured by PSA ELISA. Treatment with 0.1, 1 or 10 nM 5α-DHT, 1, 10 and 100 nM equol or a combination of 5α-DHT with equol (0.1 nM 5α-DHT and 1 nM equol; 1 nM 5α-DHT and 10 nM equol or 10 nM 5α-DHT and 100 nM equol). Cytotoxicity, as assessed by MTT Assay, did not influence PSA production by LNCAP prostate cancer cells. As shown in FIGURE 18, 1, 10 or 100 nM Vehicle PSA levels did not differ from the No Treatment baseline. Treatment with 0.1, 1 or 10 nM 5α-DHT stimulated PSA secretion to maximal levels. PSA levels from cells treated with 1, 10, and 100 nM equol were below baseline, and did not differ significantly between groups. However, combinations of 10 nM 5α-DHT and 100 nM equol, as well as 1 nM 5α-DHT and 10 nM equol, abrogated the increase in PSA secretion, compared to 5α-DHT alone. Taken together with binding demonstrated in Example 1, this Example demonstrates that equol binds the 5α-DHT molecule biologically inactivates it in skin and in the prostate.

Example 9:

This is an example of equol preventing the stimulatory effects of 5α-DHT *in vivo.* Rats were injected with 1 mg of equol for 25 consecutive days, and serum 5α-DHT levels and prostate weights were measured since it is known that the prostate is stimulated by circulating 5α-DHT. Adult (50 day-old) males (n = 16), purchased from Charles River Laboratories (Wilmington, MA, USA), were caged individually and housed in the Brigham Young University Vivarium and maintained on a 11-dark, 13-hour light schedule (lights on 0600-1900). Before purchase, the male animals were fed a diet containing approximately 200 ppm of isoflavones. At 50 days of age, upon arrival, the male rats were placed on a diet containing approximately 10 ppm of isoflavones; referred to hereafter as the Phyto-Free diet (Zeigler Bros., Gardnes, PA, USA). All animals remained on the Phyto-free diet until 215 days of age. At 150 days of age the rats were divided into two groups (control or equol treatments) that were matched by age and body weight. Starting at 190 days of age the male rats received a daily subcutaneous 0.1cc injection at the nape of the neck of vehicle DMSO) or equol at a dose of approximately 2.5 mg/kg for 25 consecutive days.

The body weights for each group were recorded weekly starting a 150 days of ago before the treatments were initiated, with weights obtained immediately before an after the treatments were administered. At 216 days of age the animals were anesthestized with Ketamine/acepromazine and blood was collected from the heart. Next the ventral prostate organ was dissected and weighed. The collected blood samples were centrifuged and serum was stored at -20° C until time of assay.

Serum testosterone, 5α-DHT, and 17β-estradiol were quantified by radioimmunoassay (RIA) kits purchased from Diagnostic System Laboratories (Webster, TX, USA). Luteininzing hormone (LH) was quantified by an assay utilizing standards from the National Institutes of Health (NIH) pituitary hormone program. The samples were run in duplicate for each RIA, with internal control samples. In all RIAs, the control values were within their normal respective ranges. The intra-assay coefficients of variance for the assays were: testosterone = 6.0 %; for 5α-dihydrotestosterone = 8%, 17β-estradiol = 5 % and LH = 9 %.

When LH and testosterone were quantified between the treatment groups there were no significant differences in these hormone levels (TABLE 6). Since LH is the gonadotrophin regulating testosterone synthesis from Leydig cells in the testes, this is not a surprising result. However, equol-injected animals displayed an approximately 50% decrease in serum 5α-DHT compared to vehicle-injected animals. Finally, when 17β-estradiol levels were determined there were no significant differences between the treatment groups. All hormone, levels were within normal ranges of that expected for this strain, age and sex of rat. Prostate weights were significantly decreased by approximately 20 % in the equol-injected males compared to control rats. This finding corresponds with the significant decrease in circulating 5α-DHT levels which are known to regulate prostatic cell proliferation, and hence, prostate weight. Thus, this *in vivo* study demonstrates that equol can contact and biologically inactive the 5α-DHT molecule as shown by the significant decrease in 5α-DHT levels in blood and significantly reduced prostate weights of equol-treated male rats. Finally, *the in vitro* and *in vivo* studies reported above demonstrates that equol would be effective in treating skin and skin diseases/disorders that are regulated by the hormone 5α-DHT.

TABLE 6. Serum 5α-DHT Levels and Prostate Weight in Equol-Treated Male Rats.

| Parameter measured | Vehicle | Equol | Change |
|---|---|---|---|
| Prostate Weight (PW), mg | 535 ± 23 | 429 ± 30* | 20%,↓ |
| PW/100g Body Weight | 76 ± 4 | 61 ± 5* | 20%↓ |
| Luteinizing Hormone (LH), ng/ml | 1.6 ± 0.2 | 1.3 ± 0.1 | NSC |
| Serum Testosterone, ng/ml | 2.1 ± 0.4 | 2.3 ± 0.5 | NSC |
| Serum 5α-DHT, pg/ml | 100 ± 18 | 52 ± 5* | 50%↓ |
| Serum 17β-Estradiol, pg/ml | 3.4 ± 0.6 | 4.8 ± 0.7 | NSC |
| n = 8 animals per treatment group. * = p < 0.05 NSC = no significant change | | | |

Example 10:

This is an example the effects of equol on stimulating collagen type 1 and III and elastin protein expression, inhibiting matrix metalloproteinase-3 (MMP-3) protein expression, inhibiting apoptosis, and stimulating cell proliferation in 3-dimensional (3-D) cultures of human dermal fibroblasts by intracellular fluorescence activated cell sorter (FACS) analysis. In this study the effects of equol on the above parameters were compared to the natural female hormone, 17β-estradiol. Both equol and 17β-estradiol were used at 10 nM concentrations which corresponds to a normal range to study using *in vitro* experiments and represent an *in vivo* concentration range of circulating 0.1 tol.0 nM 17β-estradiol and 1 nM 5α-DHT in women, and 3 nM 5α-DHT men (see Wilson JD et al, Williams Textbook of Endocrinology, 9th ed. W.B. Saunders, Philadelphia, PA, 1998).

Using an organotypic three-dimensional dermal model, which closely resembles the intact dermis and allows for tissue-like deposition and maturation of the extracellular matrix by fibroblasts, equol significantly stimulated collagen type I (FIGURE 19) and collagen type III (FIGURE 20) compared to vehicle when assayed by FACS. These studies were performed partially as a control for the use of this methodology (i.e., FACS analysis) since equol has previously been shown to increase collagen deposition by dermal monolayer fibroblasts in culture. Only equol, but not 17β-estradiol, increased both collagen types I and III. Since the net deposition of extracellular matrix molecules is also affected by the presence and activity of matrix-degrading enzymes, the expression of MMP-3/stromelysin-1 by intracellular FACS was also measured (FIGURE 21). MMP-3 is an important enzyme which can degrade collagen and elastin, as well as other extracellular matrix constituents. Both equol and 17β-estradiol reduced MMP-3 protein expression. However, equol significantly decreased the expression of MMP-3 by 3.6-fold vs. 2.3-fold for 17β-estradiol compared to vehicle. This indicates that equol is an effective agent for inhibiting the matrix-degrading enzyme that in would enhance the expression of collagen and elastin. Since elastin is an important extracellular matrix molecule, along with collagen in the maintenance of good skin health for the prevention and treatment of wrinkles, equal was tested against 17β-estradiol in the FACS analysis. In the 3-D human dermal fibroblast cultures, equol significantly stimulated elastin 2.2-fold over vehicle levels while 17β-estradiol stimulated elastin production by 1.8-fold vs. the control (FIGURE 22).

Thus, equol significantly stimulated collagen type 1, collagen type III and elastin protein expression while significantly decreasing the matrix-degrading enzyme MMP-3. As shown in FIGURE 23, 17β-estradiol significantly increases the elastin-degrading elastase enzyme expression 1.9-fold vs. vehicle levels. However, equol was not different compared to the vehicle, demonstrating that equol promotes elastin protein expression without increasing degradation of elastin.

Further FACS analysis of the same 3-D human dermal fibroblast cultures was performed by staining the cells with propidium iodide (PI) (fluorescent DNA dye). The FACS analysis via PI-stained cells was able to detect by different markers the percentage of cells that were apoptotic (engaged in programmed-cell death) and the percentage of cycling human dermal fibroblasts in S- and G2M-phases of the cell cycle. FIGURE 24 shows the cell cycle analysis for the percentage of apoptotic cells expressed in the 3-D cultures. There were no significant differences between the treatments (equol or 17β-estradiol) compared to vehicle. This demonstrates that equol or 17β-estradiol at 10 nM had no significant effect on apoptosis. However, the percentage of cycling human dermal fibroblasts in S- and G2M-phases of the cell cycle shows that equol, but not 17β-estradiol, significantly stimulated fibroblasts to proliferate at 1.5-fold compared to vehicle (FIGURE 25). This data set demonstrates that equol stimulates human dermal fibroblasts to proliferate, which is consistent with increased collagen types 1 and III and elastin protein expression in the same human dermal fibroblast cells. Furthermore, these finding correspond with the doubling of collagen deposition in human dermal fibroblasts following the application of equol.

Example 11:

This is an example the effect of equol in regulating skin tail temperature. This was tested by injecting 3 mg of equol for 5 consecutive days in adult male rats and quantifying skin tail temperatures at the end of the treatments. In this experiment rodent skin tail temperature was determine by quantifying skin temperature by sensor/radio telemetry. Male Long-Evans male rats were purchased from Charles Rivers Laboratories at 50 days of age. The animals were placed on a diet containing very low isoflavone levels (10-15 ppm of isoflavones per gram of diet) and allowed ad lib access to this diet and water throughout the experiment. At approximately 130 days of age the male rats were observed for sexual activity by mating with receptive females. All males used in this study were sexually active by mating and inseminated receptive females that in turn become pregnant and subsequently delivered normal size litters.

At approximately 160 days of age the male rats were matched by body weight and placed into either a control or equol treatment group. As this time the rats were handled for 5 to 10 minutes per day to habituate the animals to being handled. Handling consisted of placing the rat on the supine forearm surface of laboratory personnel. This handling protocol was continued each day for approximately 2 weeks or when the animal reached 174 days of age. At 175 days of the were handled as before, but 1-inch wide silk surgical tape was placed around the animal's tail approximately 1 inch from the base of the tail to simulate the placement of the temperature sensor/transponder. This handling/tape protocol took place daily from 175-180 days of age.

At 175 days of age, each animal received a 0.1 cc subcutaneous injection at the nape of the neck of either vehicle (DMSO), or a solution of 3 mg of equol. At this time the animals weighed approximately 700 grams, therefore the dose was about 4.3 mg/kg of equol per day. The treatment injections were given for 5 consecutive days.

On the $5^{th}$ day of the treatment injections (or 180 days of age) tail skin temperature was quantified by an electronic sensor/transponder taped to the base of the animal's tail. Thirty seconds after the sensor/transponder was taped to the tail the skin temperature was recorded by radiotelemetry (± 0.1 °C). The same sensor/transponder was used for all of the animals in the two treatment groups in recording skin-tail temperatures. The rats treated with equal had a 1.5°C decrease in skin temperature, compared to control rats (FIGURE 26). Control group, n = 5; equol group, n = 4, $p < 0.025$. This finding demonstrates that equol can be used to treat hot flushes associated with perimenopause symptoms or in post-menopausal women.

Example 12:

This example deomonstrates the effects of equol on skin collagen deposition by measuring Collagen Type I C-Propeptide from human dermal monolayer fibroblasts *in vitro* treated with 10 nM equol or 10 nM 17β-estradiol as in the FACS analysis experiments. There was no cytotoxicity when 10 nM equol or 10 nM 17β-estradiol was added to the tissue cultures. Treatment with 10 nM of equol significantly stimulated collagen deposition 1-8-fold compared to vehicle (FIGURE 27), while 17β-estradiol treatment was similar to that found with ascorbate treatment. This example demonstrates that 10 nM equol significantly stimulates collagen deposition by human dermal fibroblasts.

Example 13:

This is an example of the effects of vehicle (ethanol), equol-racemic or 99% S-equol as evaluated with the Irritection® Assay System in order to predict its potential to cause ocular and dermal irritation. To achieve this objective, standard volume-dependent dose-response studies were performed with the Ocular and Dermal Irritection test methods. The proprietary Ocular and Dermal Irritection assays are standardized and quantitative *in vitro* acute ocular and dermal irritation tests which utilize changes of relevant macromolecules to predict acute ocular and dermal irritancy of chemicals and chemical formulations. The Ocular and Dermal Irritection assay methods can be readily employed to evaluate multiple samples at varying volumes or concentrations and provide highly reproducible results. Thus, these tests serve as extremely useful screening tools that facilitate all stages of raw material selection, formulation development and final product selection.

The Ocular Irritection assay, depicted schematically in FIGURE 28, provides significant advances over the *in* vivo Draize test method. The Draize eye irritation assay has been criticized because of the large variability of results obtained from different laboratories that have analyzed the same specimen.

The Dermal Irritection assay, depicted schematically in FIGURE 29, is based on the principle that chemical compounds will promote measurable changes in target biomolecules and macromolecular structures. Previous studies have clearly demonstrated that the processes of protein denaturation and disaggregation that are induced in this *in vitro* assay mimic the effects that are produced when these types of irritants are applied to the skin. Consequently, this *in vitro* test may be employed to predict the *in vivo* toxic effects of chemicals and formulations.

The results of the analysis on 4% (v/v) ethanol in physiological buffered saline (PBS) vehicle, 4% (w/v) equol-racemic in vehicle and 4% (w/v) s-equol (99 % purity) in vehicle are shown below. The results of the study indicate that the sample of vehicle was classified as a borderline minimal/mild ocular irritant with an IDE score of 12.6. A similar volume-dependent dose-response study was performed with the Dermal Irritection test method. The results demonstrated that the sample was predicted to be a non-irritant with a HIE score of 0.49. TABLES 7 and 8 present a summary of results for the vehicle.

TABLE 7. Ocular Irritection - Vehicle Only

| IVI Number | Sample Description | Dose | IDE Score | Predicted Ocular Irritancy Classification |
|---|---|---|---|---|
| E7184 | VEHICLE ONLY | 25μl | 6.8 | Minimal Irritant |
| | | 50μl | 8.7 | Minimal Irritant |
| | | 75μl | 9.4 | Minimal Irritant |
| | | 100μl | *10.6* | Minimal Irritant |
| | | 125μl | 12.6[a] | Minimal/Mild Irritant |
| [a] Maximum Qualified score | | | | |

TABLE 8. Summary of the Dermal Irritection Results - Vehicle Only

| IVI Number | Sample Description | Dose | HIE Score | Predicted Dermal Irritancy Classification |
|---|---|---|---|---|
| S4243 | VEHICLE ONLY | 25μl | 0.25 | Non-Irritant |
| | | 50μl | 0.32 | Non_Irritant |
| | | 75μl | 0.41 | Non-Irritant |

(continued)

| IVI Number | Sample Description | Dose | HIE Score | Predicted Dermal Irritancy Classification |
|---|---|---|---|---|
| | | 100µl | 0.44 | Non-Irritant |
| | | 125µl | 0.49[a] | Non-Irritant |
| [a] Maximum Qualified Score | | | | |

A standard volume-dependent dose-response study was performed with the Ocular Irritection test method. The following volumes of neat sample were applied for analysis: 25, 50, 75, 100 and 125 µl. The results of the study indicated that the sample of equol racemic was classified as a mild ocular irritant with an IDE score of 14.2. A similar volume-dependent dose-response study was performed with the Dermal Irritection test method. The results demonstrated that the sample was predicted to be a non-irritant with a HIE score of 0.35. TABLES 9 and 10 present a summary of results for the 4% equol-racemic in vehicle.

TABLE 9. Ocular Irritection Results for 4% equol-racemic

| IVI Number | Sample Description | Dose | IDE Score | Predicted Ocular Irritancy Classification |
|---|---|---|---|---|
| E7185 | RACMIC EQUOL | 25µl | 7.0 | Minimal Irritant |
| | | 50µl | 9.1 | Minimal Irritant |
| | | 75µl | 11.9 | Minimal/Mild Irritant |
| | | 100µl | 12.8 | Minimal/Mild Irritant |
| | | 125µl | 14.2[a] | Mild Irritant |
| [a] Maximum Qualified Score | | | | |

TABLE10. Dermal Irritection Results for 4% equol-racemic

| IVI Number | Sample Description | Dose | HIE Score | Predicted Dermal Irritancy Classification |
|---|---|---|---|---|
| S4244 | RACEMIC EQUOL | 25µl | 0.07 | Non-Irritant |
| | | 50µl | 0.15 | Non-Irritant |
| | | 75µl | 0.24 | Non-Irritant |
| | | 100µl | 0.30 | Non-Irritant |
| | | 125µl | 0.35[a] | Non-Irritant |
| [a] Maximum Qualified Score | | | | |

TABLES 11 and 12 present a summary of results for 4% s-equol (w/v) in vehicle. A standard volume-dependent dose-response study was performed with the Ocular Irritection test method. The following volumes of neat sample were applied for analysis: 25, 50, 75, 100 and 125 µl. The results of the study indicated that the sample of s-equol was classified as a mild ocular irritant with an IDE score of 16.4. A similar volume-dependent dose-response study was performed with the Dermal Irritection test method. The results demonstrated that the sample was predicted to be a non-irritant with a HIE score of 0.15. The raw test materials: ethanol vehicle, equol-racemic and S-equol were analyzed in the Ocular and Dermal Irritection tests at 4 percent. Since the vehicle results displayed similar values to that of the equol results (racemic equol or S-equol), this demonstrates that most of the irritant effects seen in the equol samples can be attributed to the vehicle. Therefore, for ocular and dermal applications, racemic equol and S-equol are classified as non-irritating based upon the results for skin/cosmetic relevance.

TABLE 11. Ocular Irritection Results for 4 % s-equol

| IVI Number | Sample Description | Dose | IDE Score | Predicted Ocular Irritancy Classification |
|---|---|---|---|---|
| E7186 | S-EQUOL | 25μl | 12.9 | Minimal/Mild Irritant |
| | | 50μl | 13.5 | Minimal/Mild Irritant |
| | | 75μl | 14.2 | Mild Irritant |
| | | 100μl | 14.7 | Mild Irritant |
| | | 125μl | 16.4[a] | Mild Irritant |
| [a] Maximum Qualified Score | | | | |

TABLE 12. Dermal Irritection Results for 4 % s-equol

| IVI Number | Sample Description | Dose | HIE Score | Predicted Dermal Irritancy Classification |
|---|---|---|---|---|
| S4245 | S-EQUOL | 25μl | 0.06 | Non-Irritant |
| | | 50μl | 0.08 | Non-Irritant |
| | | 75μl | 0.11 | Non-Irritant |
| | | 100μl | 0.13 | Non-Irritant |
| | | 125μl | 0.15[a] | Non-Irritant |
| [a]Maximum Qualified Score | | | | |

The *in viva* and *in vitro* examples presented herein demonstrate that equol has the ability to bind 5α-DHT and biologically inactivate its hormonal influence in skin, hair and prostate, and to stimulate skin cells to proliferate and produce collagen type 1, collagen type III and elastin protein expression, all of which contribute to improved skin/hair health and treatment applications of androgen-dependent diseases/disorders of the skin/hair.

While various embodiments of the present invention have been described in detail, it will be apparent that further modifications and adaptations of the invention will occur to those skilled in the art. It is to be expressly understood that such modifications and adaptations are within the spirit and scope of the present invention.

The invention to the aspect as defined in the following numbered paragraphs:

1. A method of co-mediating androgen hormone action and estrogen hormone action, that ameliorate one or more physiological and pathophysiological conditions/disorders of the skin in human and non-human species, by administering an enantiomeric equol comprising S-equol, in an amount sufficient to bind free 5α-dihydrotestosterone and inhibit its binding with androgen receptors, and to bind estrogen receptor subtypes.

2. The method according to paragraph 1 further comprising R-equol.

3. A method of mediating androgen hormone action that ameliorates one or more physiological and pathophysiological conditions/disorders of the skin in human and non-human species, by administering an enantiomeric equol comprising R-equol, in an amount sufficient to bind free 5α-dihydrotestosterone and inhibit its binding with androgen receptors.

4. The method according to paragraph 3 wherein the amount of equol administered is sufficient to bind estrogen receptors.

5. The method according to paragraph 1 or 3, wherein the skin condition/disorder is selected from the group consisting of: skin integrity, collagen production, elastin production, elastase, skin thickness, blood flow in the skin, skin turgor, skin moisture content, vaginal dryness, prevention of collagen and elastin breakdown by matrix metalloproteinases, repair and prevention of wrinkles in skin, enhancing glycoaminoglycans and hyaluronic acid for improved skin appearance, wound healing, improvement of scars in skin, decrease oily skin by improving sebaceous gland function, skin age spots and skin lightening, acne, male and female pattern baldness, hirsutism, scalp, facial and body hair health and growth, apocrine (sweat) gland function, inflammation of the skin, immune function in the skin, skin pore size, skin temperature and skin and hair abnormalities in steroid hormone synthesis/hormone action, metabolism of steroids and binding steroid receptors involving androgenic and/or estrogenic effects.

6. The method according to pargraph 1 or 3, wherein the method of administering comprises administration of the equol at a tissue site.

7. The method according to paragraph 1 or 3 wherein the step of administering equol decreases matrix metalloproteinases (MMPs) and prevents the breakdown of collagen in the skin.

8. The method according to paragraph 1 or 3 wherein the step of administering equol, enhances blood flow and vascular endothelial growth factor in the skin.

9. The method according to paragraph 1 or 3 wherein the step of administering equol improves scalp hair follicle health, scalp hair growth, enhances scalp hair health and scalp hair color in non-balding and balding individuals.

10. The method according to paragraph 1 or 3 wherein the step of administering equol decreases the amount of sweating or perspiration from apocrine glands (in general) and apocrine glands associated with hairy regions of the body such as armpits and groin areas.

11. The method according to paragraph 1 or 3 wherein the step of administering equol comprises the step of contacting the skin of the individual with a substrate comprising an effective amount of equol contained thereon.

12. The method according to paragraph 1 or 3 wherein the step of administering equol decreases skin temperature such as in hot flushes in women.

13. The method according to paragraph 1 or 3 wherein the step of administering equol improves skin cancerous conditions, such as malignant melanomas.

14. The method according to paragraph 1 or 3 wherein the equol is administered as an oral composition comprising at least 0.01 mg enantiomeric equol per kg body weight.

15. The method according to paragraph 1 or 3 wherein the equol is administered as a topical composition comprising at least 0.01 %, and up to about 10%, Enantiomeric equol.

16. The method according to paragraph 1 or 3 wherein the equol is administered via a product form comprising equol, selected from the group consisting of a lotion, a spray solution, a pad, a bandage, or a transdermal patch.

17. A method of treating and preventing androgen-related diseases mediated by androgen hormone action, by administering an enantiomeric equol comprising S-equol, in an amount sufficient to bind free 5$\alpha$-dihydrotestosterone and inhibit its binding with androgen receptors, and to bind estrogen receptor subtypes.

18. A method of treating and preventing androgen-related diseases mediated by androgen hormone action, by administering an Enantiomeric equol comprising R-equol, in an amount sufficient to bind free 5$\alpha$-dihydrotestosterone and inhibit its binding with androgen receptors.

19. The use of an enantiomers of equol comprising S-equol, for treating and preventing androgen-related diseases mediated by androgen hormone action, by administering an enantiomeric equol comprising S-equol, in an amount sufficient to bind free 5$\alpha$-dihydrotestosterone and inhibit its binding with androgen receptors, and to bind estrogen receptor subtypes.

20. A method of providing a personalized treatment of one or more physiological and pathophysiological conditions/disorders of the skin in human and non-human species, mediated both by DHT and the estrogen receptors, comprising: 1) assessing the one or more disease states or conditions of a patient; 2) assessing the equol-producer status of the patient; 3) determining an optimally beneficial course of treatment, selected from the group consisting of a) a mode of administration, b) a dose amounts, c) a dose interval, and d) the enantiomeric ratio of the equol dose.

**Claims**

1. Enantiomeric equol comprising R-equol in an amount sufficient to bind free 5α-dihydrotestosterone and inhibit its binding with androgen receptors for use improving the blood flow in the skin, enhancing wound healing, and/or improving apocrine gland function of the skin.

2. The enantiomeric equol for use according to claim 1, wherein the equol is a racemic or non racemic mixture of R-equol and S-equol.

3. The Enantiomeric equol for use according to claim 1 or claim 2, wherein the equol is sufficient to bind estrogen receptors.

4. The Enantiomeric equol according to claim 1 or claim 2, for use in enhancing blood flow in the skin.

5. The enantiomeric equol for use according to claim 1 or claim 2, wherein the enantiomeric equol is formulated for administration at a tissue site.

6. The Enantiomeric equol for use according to claim 1 or claim 2, wherein the equal is formulated to be contacted with the skin of an individual.

7. The enantiomeric equol for use according to claim 1 or claim 2, wherein the equol is formulated as a topical composition comprising at least 0.01 % and up to 10% enantiomeric equol.

8. The Enantiomeric equol for use according to claim 1 or claim 2, wherein the equol is administered as an oral composition comprising at least 0.01mg equol per kg body weight.

9. The Enantiomeric equol for use according to claim 1 or claim 2, wherein the equol is formulated as a lotion, a spray solution, a pad, a bandage, or a transdermal patch.

10. A method for ameliorating a skin condition selected from the group consisting of improving skin turgor, preventing collagen and elastin breakdown by matrix metalloproteinases, enhancing elastin production; wherein said method comprises administering Enantiomeric equal comprising R-equol in an amount sufficient to bind free 5α-dihydrotestosterone.

11. The method according to claim 10, wherein the equol is a racemic or non racemic mixture of R-equol and S-equol.

12. The method according to claim 10 or claim 11, wherein the equol is administered at a level sufficient to bind estrogen receptors.

13. The method according to claim 10 or claim 11, wherein an effective amount of equol is formulated to be contacted with the skin of an individual.

14. The method according to claim 10 or claim 11, wherein the equol is formulated as a topical composition comprising at least 0.01 % and up to 10% enantiomeric equol,

15. The method according to claim 10 or claim 11, wherein the equol is formulated as a lotion, a spray solution, a pad, a bandage, or a transdermal patch.

Figure 1

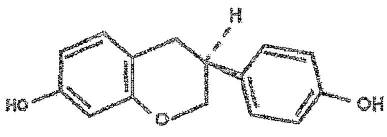

S-equol

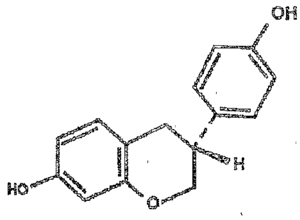

R-equol

Figure 2

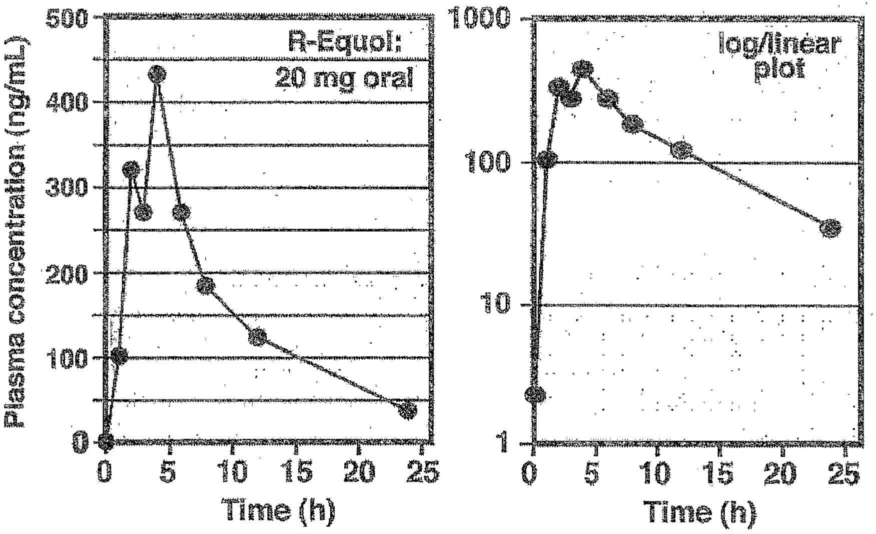

Figure 3

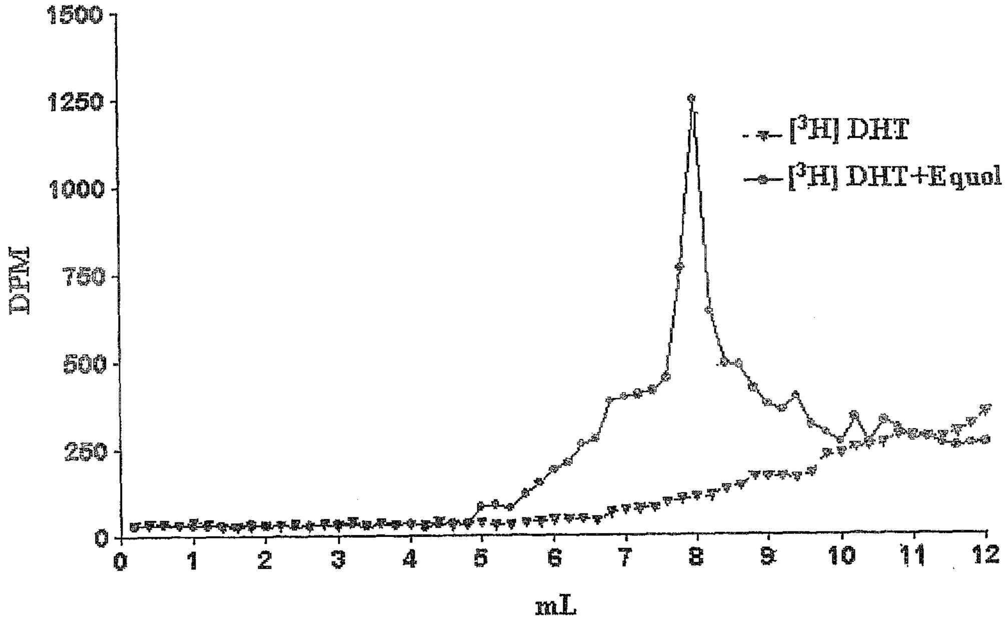


Figure 5

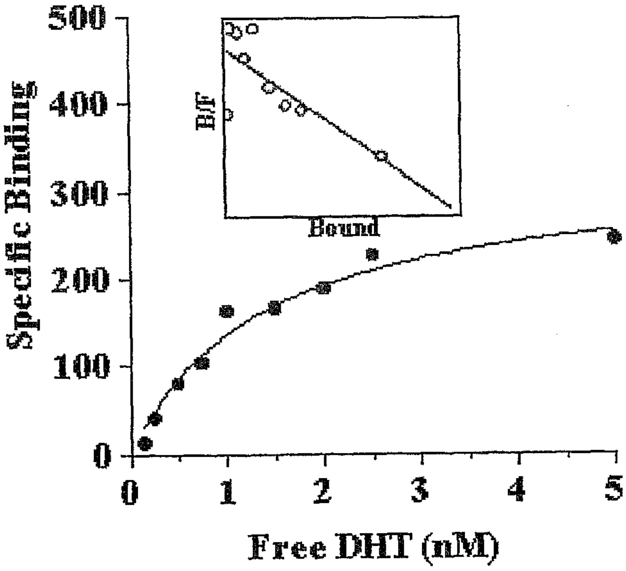

Figure 4A

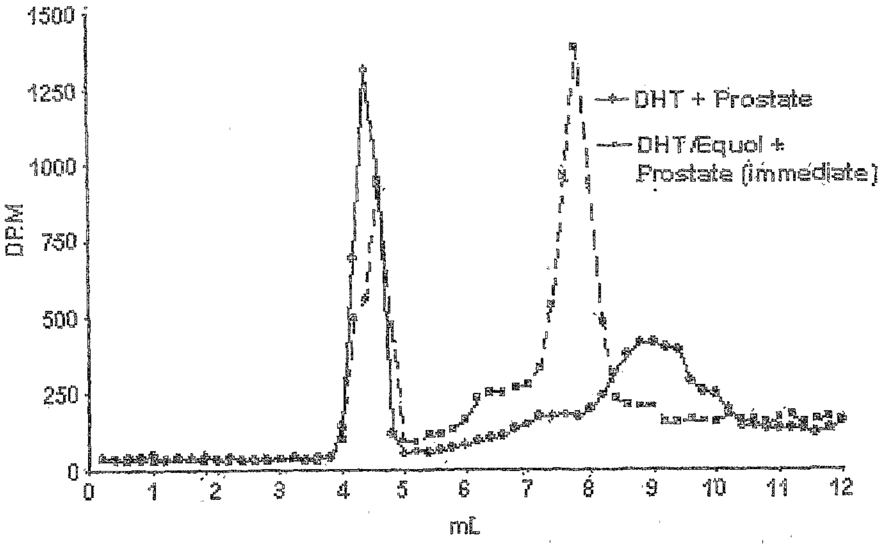


Figure 4B

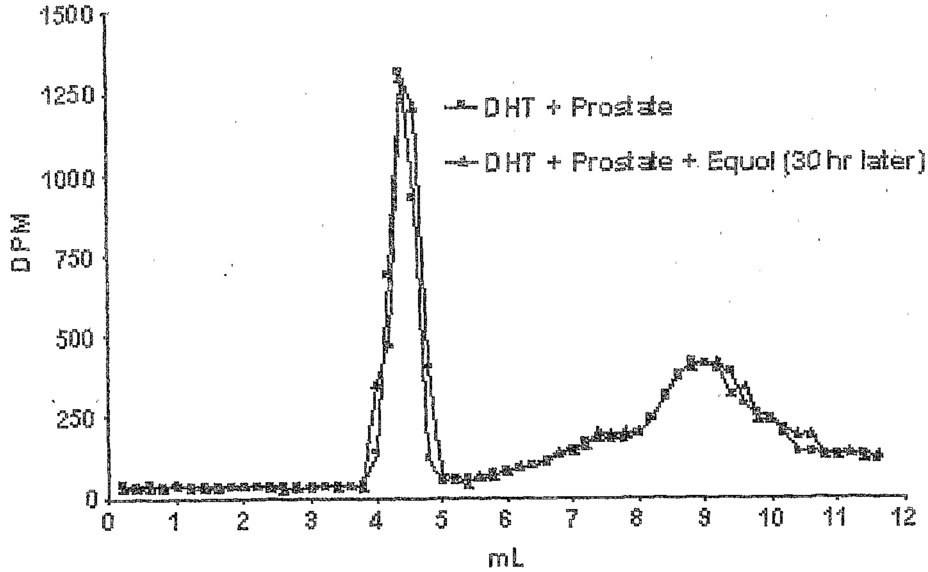

Figure 6

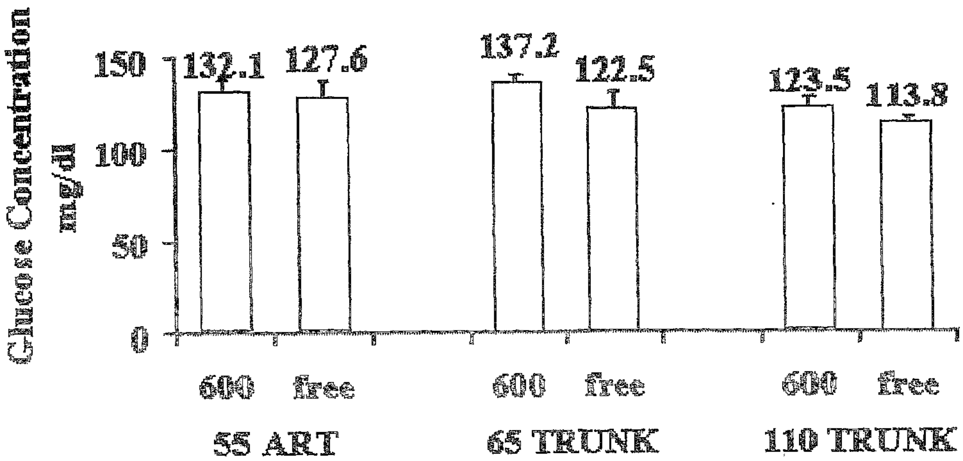


Figure 7

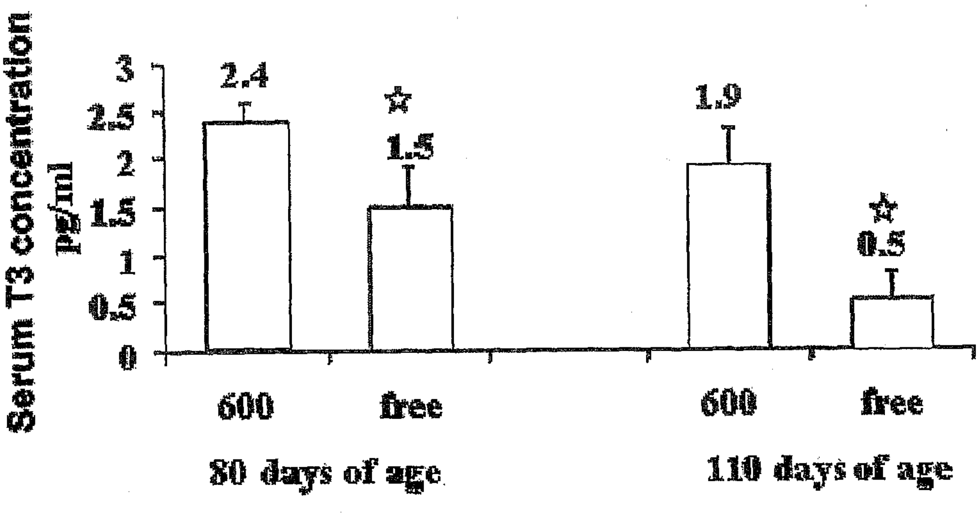

Figure 8

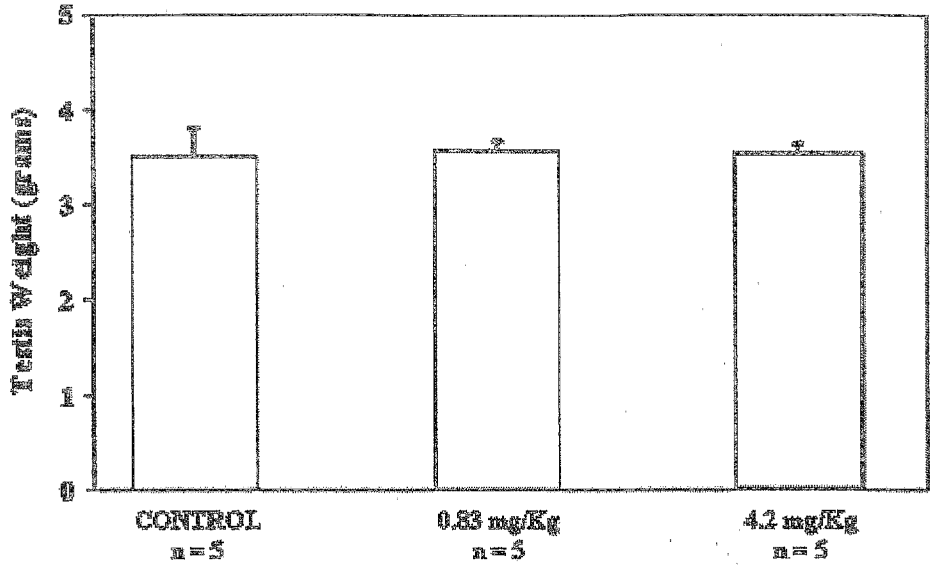


Figure 9A

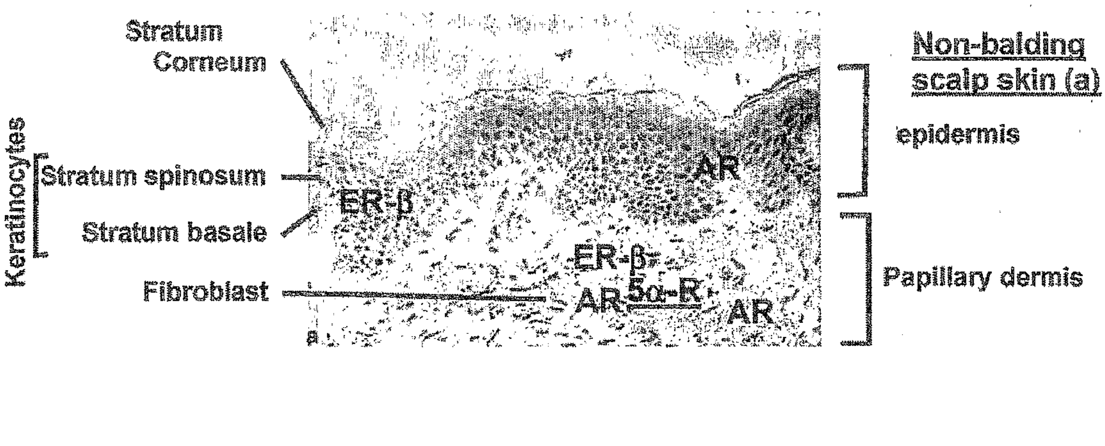

Figure 9B

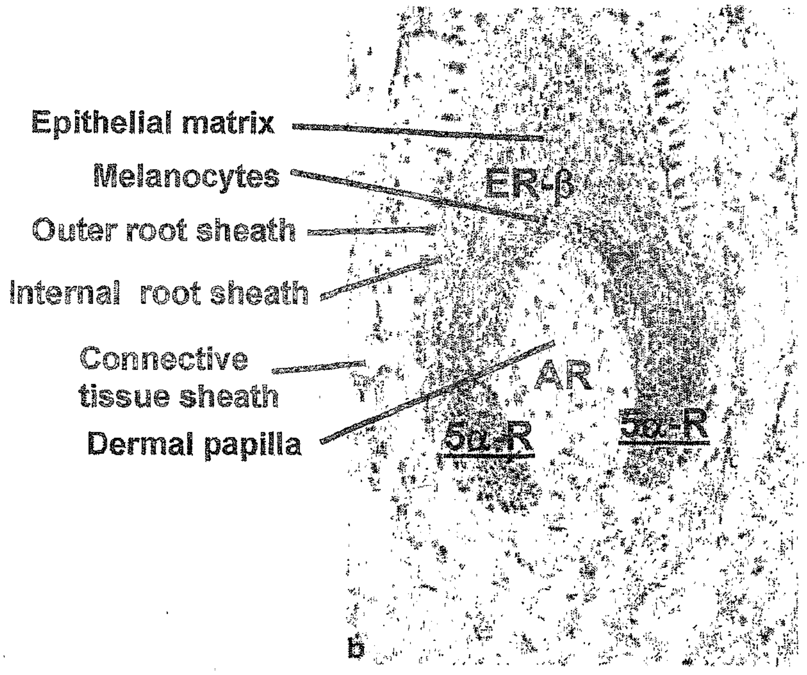


Figure 9C

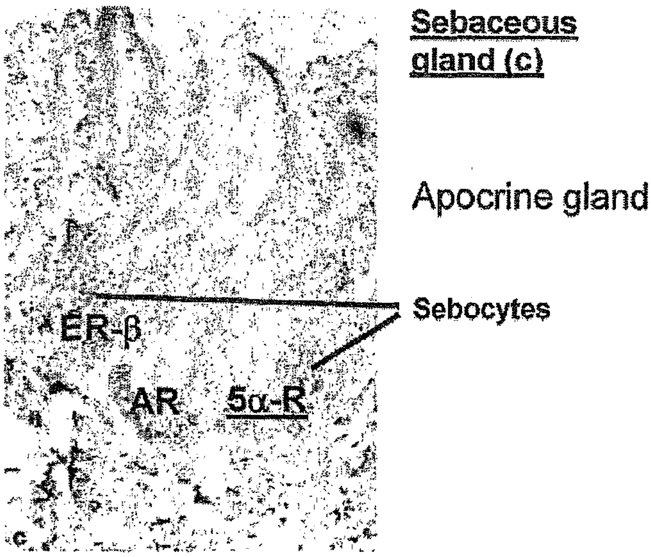

Figure 10

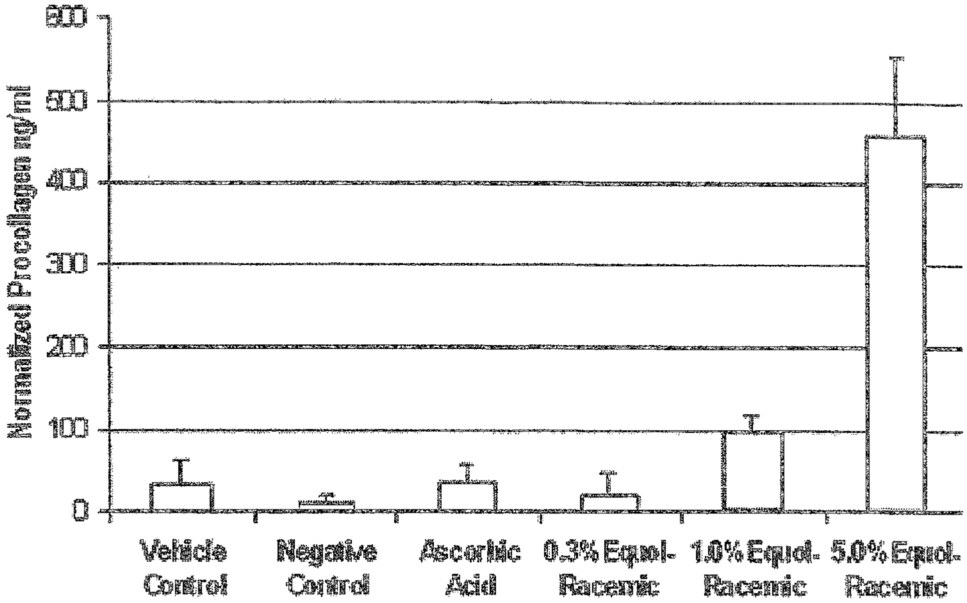


Figure 11

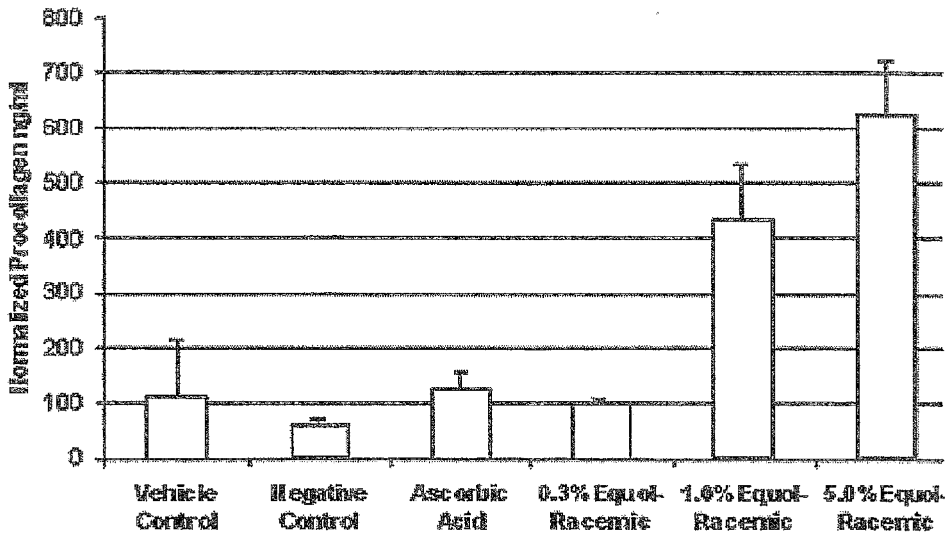

Figure 12

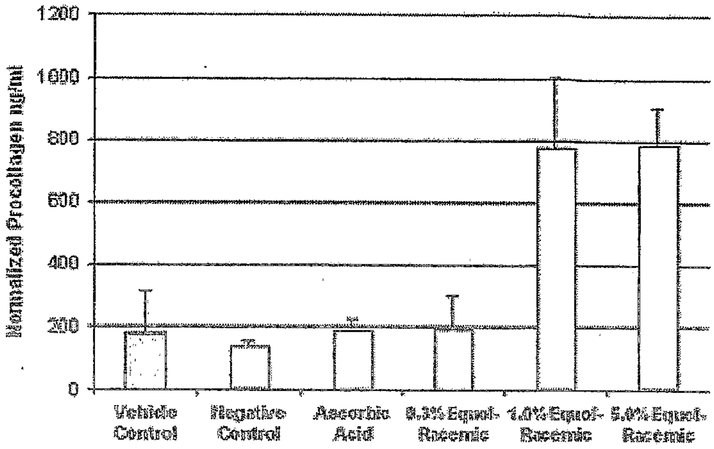


Figure 13

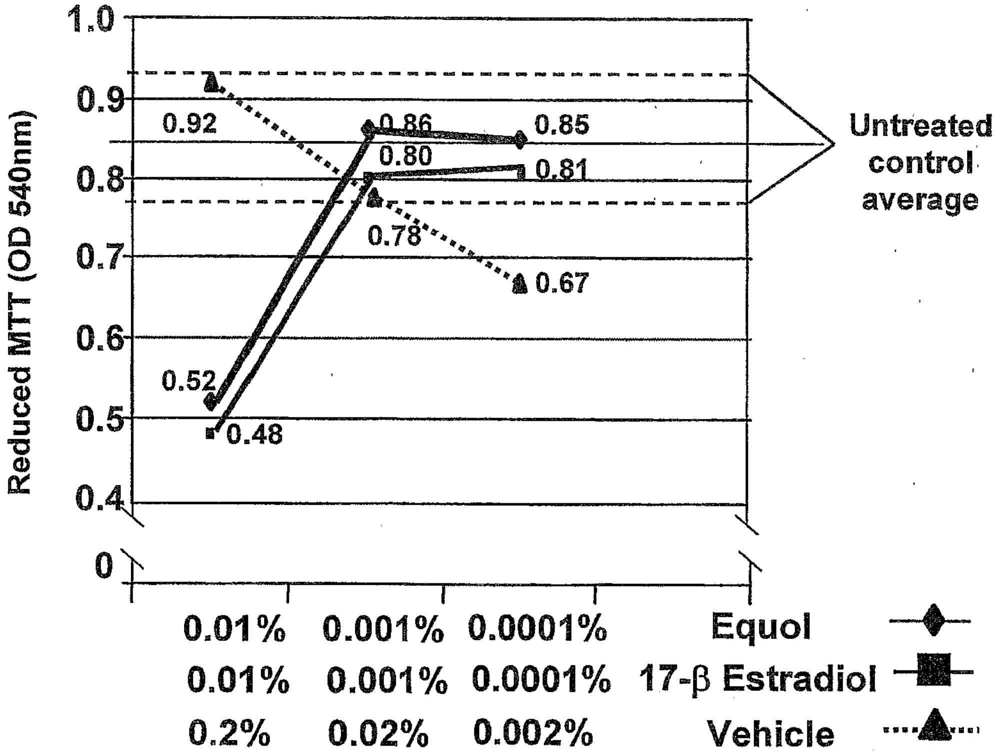

Figure 14

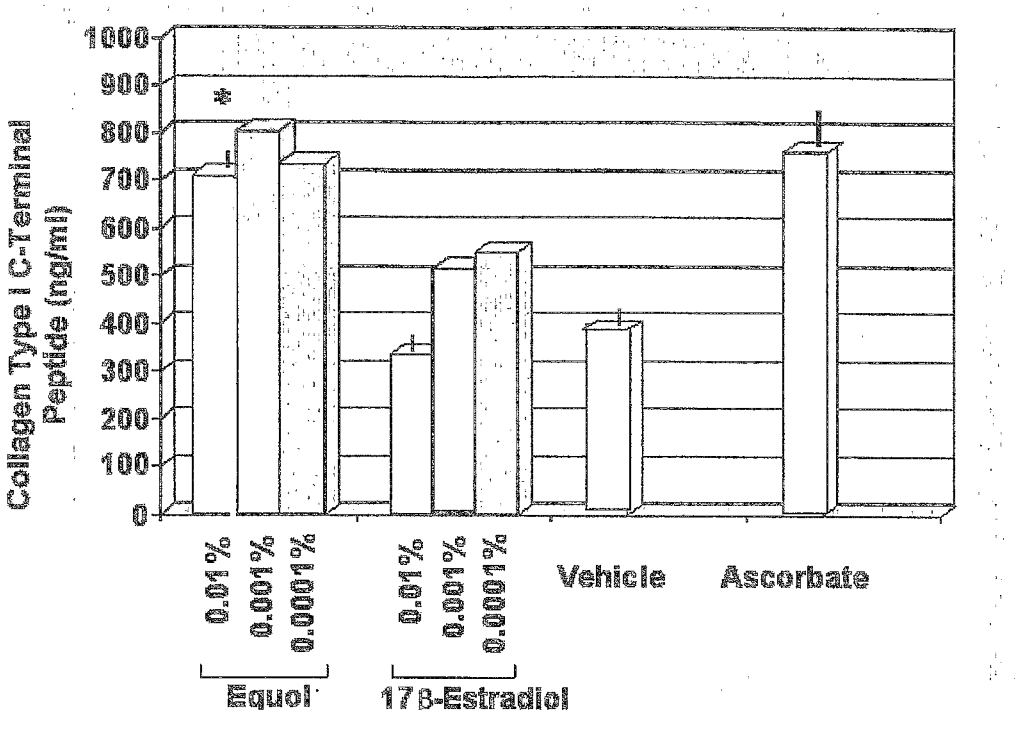
Collagen Type I C-Terminal Peptide (ng/ml)
1000
900
800
700
600
500
400
300
200
100
0
*
0.01%
0.001%
0.0001%
0.01%
0.001%
0.0001%
Vehicle
Ascorbate
Equol
17β-Estradiol

Figure 15

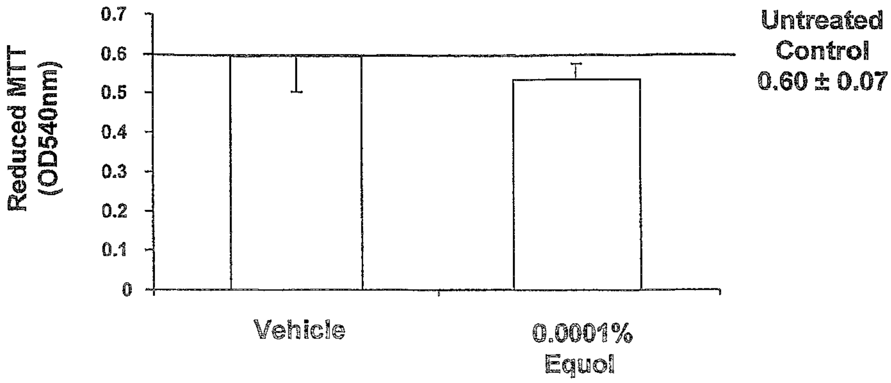


Figure 16

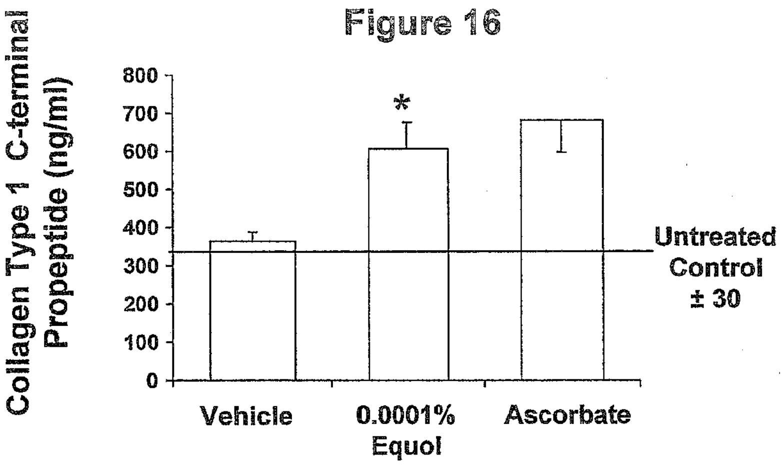


Figure 17

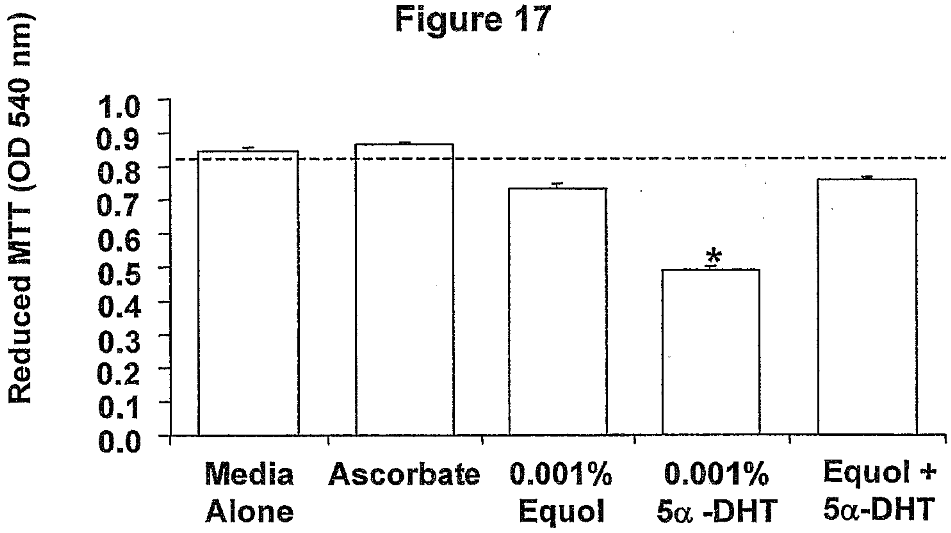

Figure 18

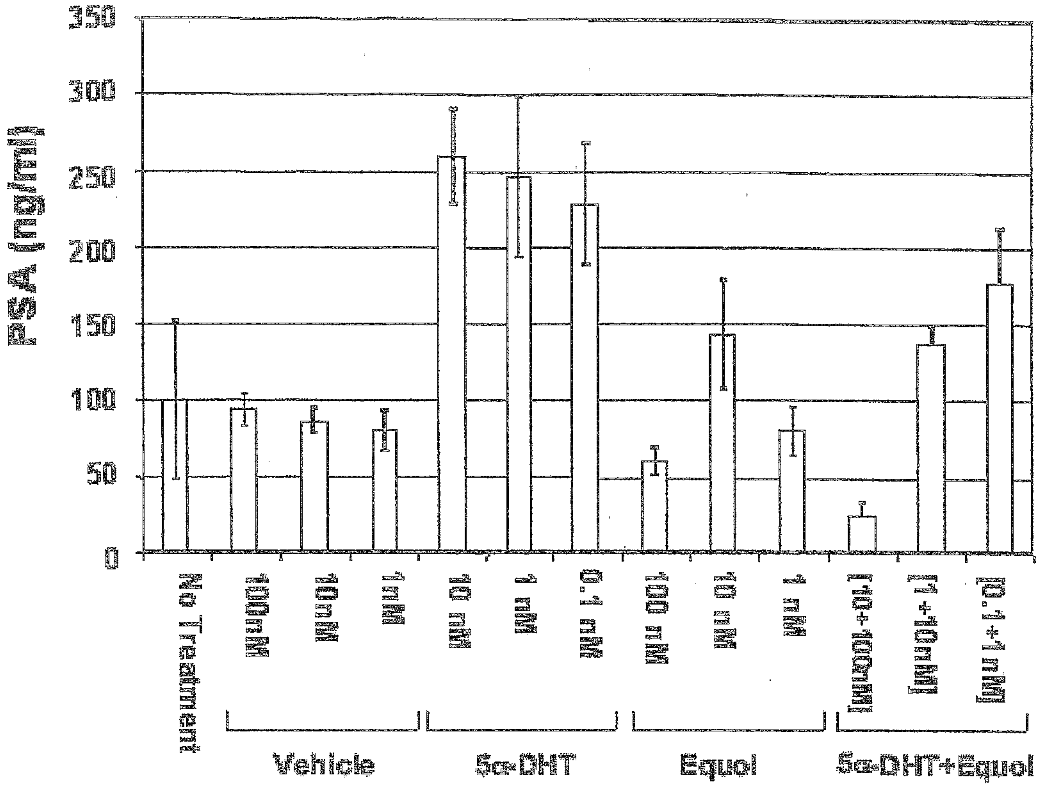


Figure 19

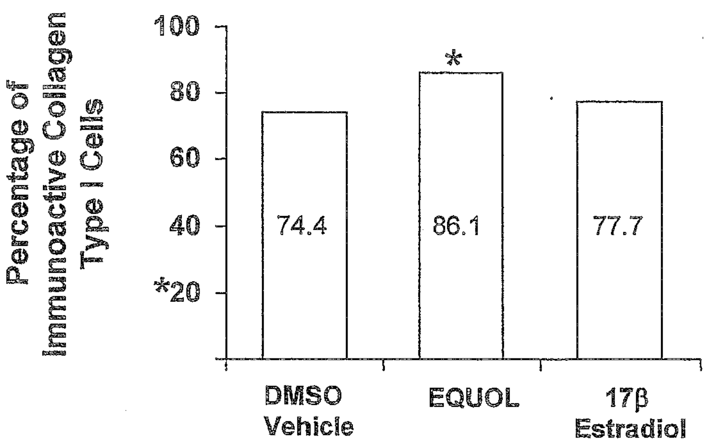

Figure 20

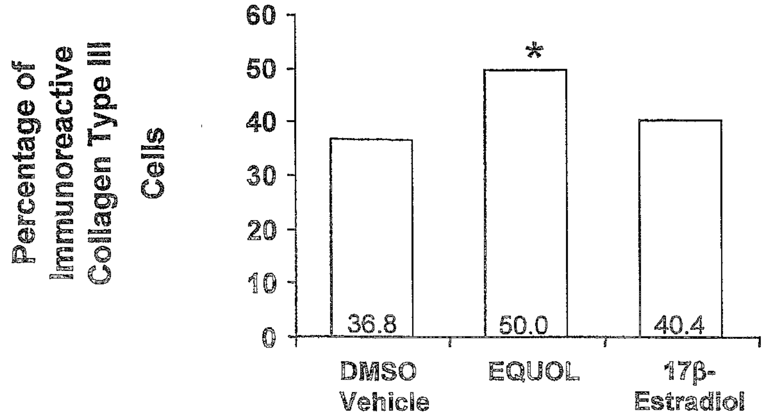


Figure 21

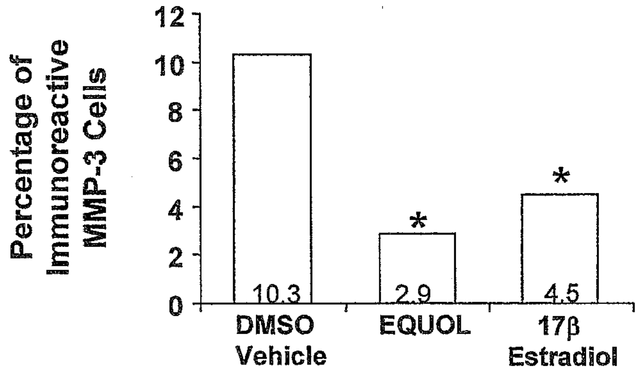


Figure 22

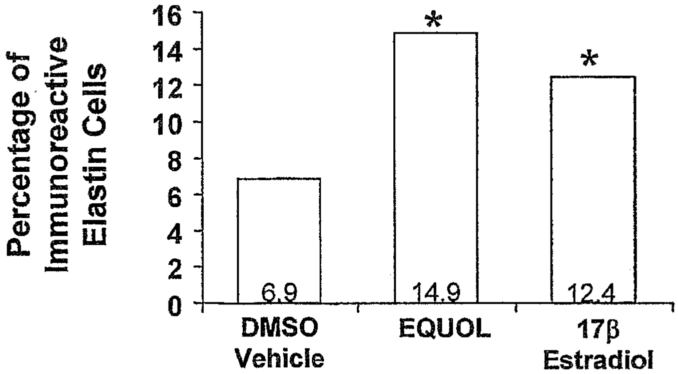

Figure 23

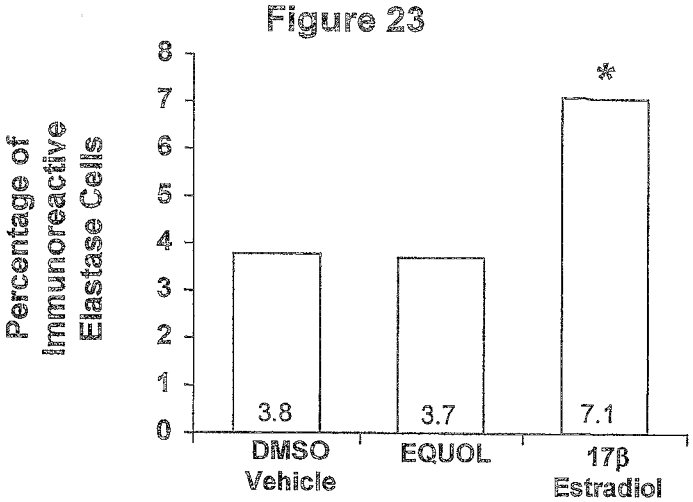


Figure 24

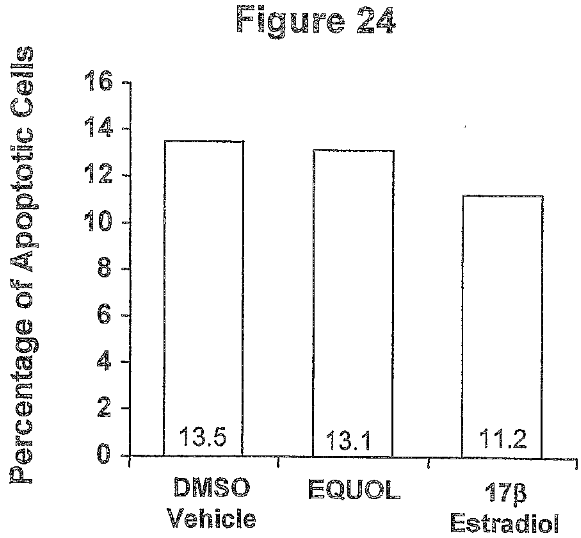


Figure 25

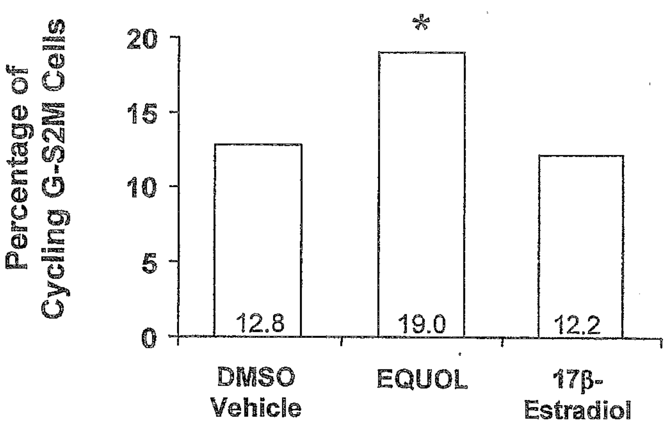

Figure 26

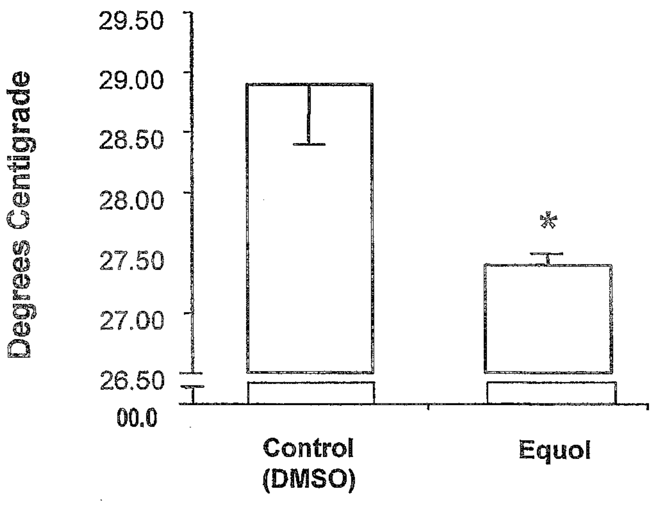


Figure 27

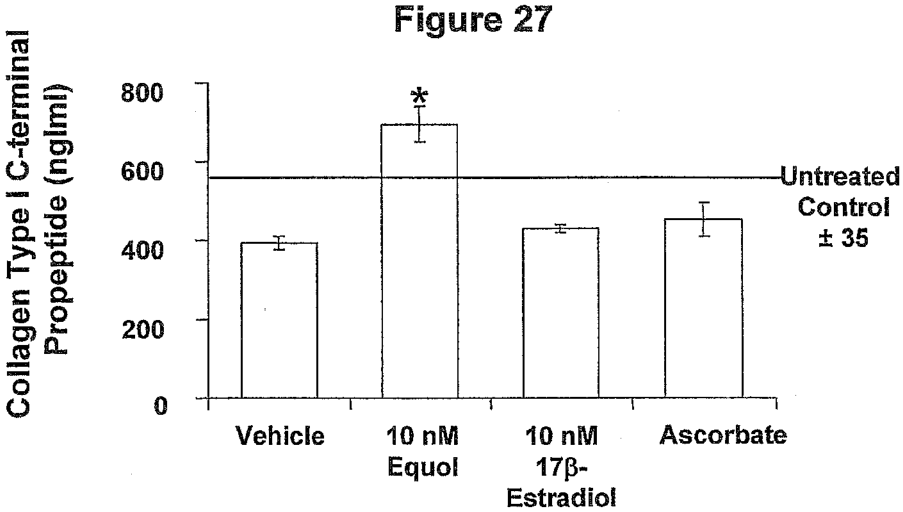

Figure 28

Ocular Irritection Model

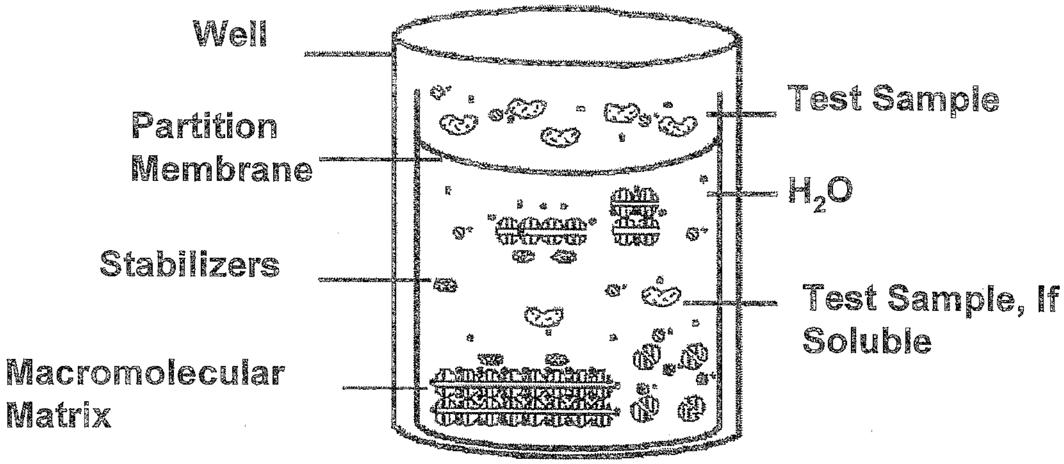


Figure 29

Dermal Irritection Model

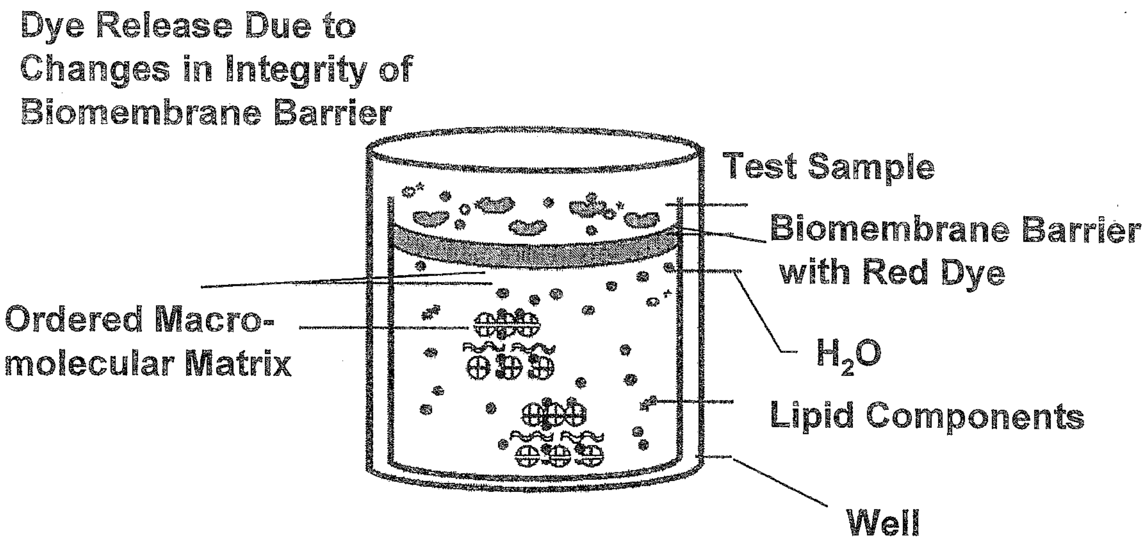

Europäisches Patentamt
European Patent Office
Office européen des brevets

# EUROPEAN SEARCH REPORT

Application Number
EP 10 19 4495

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | WO 2004/009035 A2 (CHILDRENS HOSP MEDICAL CENTER [US]; SETCHELL KENNETH DAVID REGINAL [US) 29 January 2004 (2004-01-29) * paragraphs [0001], [0018], [0019], [0119], [0140], [0142] * ----- | 1-9 | INV. A61K31/7048 A61K31/353 A61K47/00 A61P17/00 |
| X | WO 98/56373 A (GORBACH SHERWOOD L [US]) 17 December 1998 (1998-12-17) * page 2, line 3 - line 10 * * claim 2 * ----- | 10-15 | |
| X,P | WO 2004/039327 A (UNIV COLORADO RES [US]; UNIV BRIGHAM YOUNG [US]; CHILDRENS HOSP MEDICA) 13 May 2004 (2004-05-13) * paragraphs [0094], [0122], [0127], [0139]; claims 7-9 * ----- | 10-15 | |
| A | WO 2004/026274 A (NOVOGEN RES PTY LTD [AU]; KELLY GRAHAM EDMUND [AU]; HUSBAND ALAN [AU];) 1 April 2004 (2004-04-01) * claims * ----- | 1-15 | TECHNICAL FIELDS SEARCHED (IPC) |
| A | WO 02/067988 A (UNIV MICHIGAN [US]) 6 September 2002 (2002-09-06) * claims * ----- | 1-15 | A61K A61P |
| A | WO 02/087517 A (BEIERSDORF AG [DE]; BIERGIESSER HELGA [DE]; DOERING THOMAS [DE]; GALLI) 7 November 2002 (2002-11-07) * the whole document * ----- | 1-15 | |
| A | WO 00/13661 A (AVON PROD INC [US]; DURAISWAMI CHAYA [US]; SIMPSON SUSAN E [US]; GARRI) 16 March 2000 (2000-03-16) * claims * ----- | 1-15 | |

The present search report has been drawn up for all claims

1

| Place of search | Date of completion of the search | Examiner |
|---|---|---|
| Munich | 22 February 2011 | Loher, Florian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 19 4495

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2011

| Patent document cited in search report | Publication date | Patent family member(s) | Publication date |
|---|---|---|---|
| WO 2004009035 A2 | 29-01-2004 | AU 2003259220 A1 | 09-02-2004 |
| | | BR 0313182 A | 24-07-2007 |
| | | CA 2492754 A1 | 29-01-2004 |
| | | CN 1681386 A | 12-10-2005 |
| | | CN 101965974 A | 09-02-2011 |
| | | EP 1545206 A2 | 29-06-2005 |
| | | JP 2006504409 T | 09-02-2006 |
| | | JP 2010143930 A | 01-07-2010 |
| | | US 2009018185 A1 | 15-01-2009 |
| | | US 2010120705 A1 | 13-05-2010 |
| | | US 2004235758 A1 | 25-11-2004 |
| | | US 2004147594 A1 | 29-07-2004 |
| WO 9856373 A | 17-12-1998 | AU 7694298 A | 30-12-1998 |
| | | CA 2294062 A1 | 17-12-1998 |
| | | EP 0998262 A1 | 10-05-2000 |
| | | JP 2002511860 T | 16-04-2002 |
| | | US 6060070 A | 09-05-2000 |
| WO 2004039327 A | 13-05-2004 | AU 2003286781 A1 | 25-05-2004 |
| | | CA 2504682 A1 | 13-05-2004 |
| | | EP 1569636 A2 | 07-09-2005 |
| | | JP 2006508942 T | 16-03-2006 |
| WO 2004026274 A | 01-04-2004 | CA 2499602 A1 | 01-04-2004 |
| | | CZ 20050181 A3 | 17-08-2005 |
| | | EP 1542654 A1 | 22-06-2005 |
| | | JP 2006508058 T | 09-03-2006 |
| | | MX PA05003202 A | 05-07-2005 |
| | | NZ 539149 A | 31-05-2007 |
| | | US 2006153782 A1 | 13-07-2006 |
| WO 02067988 A | 06-09-2002 | BR 0207663 A | 25-10-2005 |
| | | CA 2439408 A1 | 06-09-2002 |
| | | EP 1370294 A2 | 17-12-2003 |
| | | JP 2005506285 T | 03-03-2005 |
| | | JP 2009079038 A | 16-04-2009 |
| | | MX PA03007609 A | 30-06-2004 |
| WO 02087517 A | 07-11-2002 | DE 10121375 A1 | 07-11-2002 |
| | | EP 1392239 A2 | 03-03-2004 |
| WO 0013661 A | 16-03-2000 | AU 6034599 A | 27-03-2000 |
| | | BR 9906998 A | 26-09-2000 |
| | | CA 2309179 A1 | 16-03-2000 |
| | | EP 1041964 A1 | 11-10-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 04009035 PCT
- US 5525346 A
- US 5613958 A
- US 6071531 A
- US 5693018 A
- US 5848991 A
- US 5468501 A


### Non-patent literature cited in the description


- **KNIGHT et al.** *Obstet Gyneco,* 1996, vol. 187, 897-904
- **SETCHELL, KDR.** *Am J Clin Nutr,* 1998, vol. 129, 1333S-1346S
- **KURZER et al.** *Annu Rev Nutr,* 1997, vol. 17, 353-381
- **SETCHELL et al.** *The Lancet,* 1997, vol. 350, 23-27
- **PELLETIER ; REN.** *Histol Histopathology,* 2004, vol. 19, 629-636
- **BRINCAT MP.** *Maturitas,* 2000, vol. 29, 107-117
- **PUNNONEN R.** *Acta Obstet Gynecol Scand,* 1972, vol. 21, 3-44
- **HASSELQUIST, MB et al.** *J Clin Endocrinol Metab,* 1980, vol. 50, 76-82
- **SHAH MG ; MAIBACH HI.** *Am J Clin Dermatol,* 2001, vol. 2, 143-150
- **PIRILA E et al.** *Curr Med Chem,* 2001, vol. 8, 281-294
- **RAINE-FENNING NJ et al.** *Am J Clin Dermatol,* 2003, vol. 4, 371-378
- **ASHCROFT GE et al.** *Nat Med,* 1997, vol. 3, 1209-1215
- **THORNTON MJ et al.** *Exp Dermatol,* 2003, vol. 12, 181-190
- **THORNTON MJ et al.** *J Invest Dermatol Symp Proc,* 2003, vol. 8, 100-103
- **NITSCH SM et al.** *Arch Surg,* 2004, vol. 139, 157-163
- **GILLIVER SC et al.** *Thromb Haemost,* 2003, vol. 90, 978-985
- **REED MJ ; FRANKS S.** *Baillieres Clin Obstet Gynaecol,* 1988, vol. 2, 581-595
- **TRUEB RM.** *Exp Gerontol,* 2002, vol. 37, 981-990
- **LACHGAR S et al.** *J Invest Dermatol Symp Proc,* 1999, vol. 4, 290-295
- **POCHI PE ; STRAUSS JS.** *J Invest Dermatol,* 1974, vol. 62, 191-210
- **LARIE F et al.** *Horm Res,* 2000, vol. 54, 218-229
- **JAKUBOVIC HR et al.** *Dermatology,* 1992, 69-77
- **SATO T et al.** *Br J Dermatol,* 1998, vol. 139, 806-810
- **OFFIDANI A et al.** *J Clin Pathol,* 1999, vol. 52, 829-832
- **WADE TR et al.** *Obstet Gynecol,* 1978, vol. 52, 233-242
- **TOBIN DJ ; BYSTRYN JC.** *Pigment Cell Res,* 1996, vol. 9, 304-310
- **THORTON MJ.** *Exp Dermatology,* 2002, vol. 11, 487-502
- **OHUCHI A et al.** *Third Intercontinental Meeting of Hair Research Societies, Japan,* 2001
- **KANDA N ; WATANABE S.** *J Invest Dermatol,* 2001, vol. 117, 274-283
- **RICHARDSON B et al.** *Br J Cancer,* 1999, vol. 80, 2025-2033
- **DURVASULA R et al.** *Climacteric,* 2002, vol. 5, 1970200
- Handbook of Pharmaceutical Excipients. American Pharmaceutical Association, 1994
- **FLEISHMAJER.** *J Invest Dermatol,* 1991, vol. 97, 638-643
- **CONTARD.** *Cell Tissue Res,* 1993, vol. 273, 571-575
- **PINNEY ; LIU ; SHEEMAN ; MANSBRIDGE.** *J Cell Physiology,* 2000, vol. 183, 74-82
- **SLIVKA.** *J Invest Dermatol,* 1993, vol. 100, 40-46
- **K.D.R. SETCHELL.** *Am J Clin Nutr,* 1998, vol. 129, 1333S-1346S
- **K.D.R. SETCHELL et al.** *J Nutr,* 2002, vol. 132, 3577-3584
- **WATANABE, S. et al.** *Biofactors,* 2000, vol. 12, 233-41
- **LEPHART, E.D. et al.** *Nutrition Metab (London,* 2004, vol. 1, 16

- Final report on the safety, assessment of butylenes glycol, hexylene glycol, ethoxydiglycol, and dipropylene glycol. Journal of the American College of Toxicology. Mary Ann Liebert, Inc. Publishers, 1985, vol. 4
- **WILSON JD et al.** Williams Textbook of Endocrinology. W.B. Saunders, 1998